(19) Europäisches Patentamt
European Patent Office
Office européen des brevets

(11) EP 4 046 487 A1

(12) EUROPEAN PATENT APPLICATION

(43) Date of publication:
24.08.2022 Bulletin 2022/34

(21) Application number: 22158322.2

(22) Date of filing: 23.02.2022

(51) International Patent Classification (IPC):
A01M 21/04 (2006.01)

(52) Cooperative Patent Classification (CPC):
A01M 21/04; A01M 21/043; A01M 21/046

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 23.02.2021 GB 202102568

(71) Applicants:
• Bardos, Richard Paul
Reading, Berkshire RG4 8NN (GB)
• Layland, David Andrew
Stockport, Cheshire SK4 4AN (GB)

• Podmore, Richard
Stockport, Cheshire SK8 7EH (GB)

(72) Inventors:
• Bardos, Richard Paul
Reading, Berkshire RG4 8NN (GB)
• Layland, David Andrew
Stockport, Cheshire SK4 4AN (GB)
• Podmore, Richard
Stockport, Cheshire SK8 7EH (GB)

(74) Representative: Abel & Imray LLP
Westpoint Building
James Street West
Bath BA1 2DA (GB)

(54) METHODS FOR DETERMINING THE EFFECTIVENESS OF PLANT CONTROL TREATMENT

(57) A method of determining the effectiveness of a lethal treatment applied to a plant or a group of two or more plants is disclosed. The method comprises assessing whether the treated plant or plants is dead on the basis of a comparative electrolyte leakage value of one or more root or rhizome samples taken from the treated plant or group of plants. A method of treating a plant or group of plants with a lethal treatment, a method of validating a treatment for killing a plant, and a method of optimising a heat treatment for killing a plant are also disclosed. Further disclosed are computer-implemented methods of obtaining or making an assessment of the effectiveness of a lethal treatment which has been applied to a plant or group of two or more plants.

Fig. 2

**Description**

The present invention concerns methods for determining the effectiveness of a lethal treatment applied to one or a group of two or more plants. More particularly, but not exclusively, this invention concerns methods for determining the effectiveness of a lethal treatment applied to one or a group of two or more plants, wherein if a sample of the one or more treated plants is determined to be alive, then the lethal treatment is assessed to have been unsuccessful, whereas if a sample of the one or more treated plants is determined to be dead, then the lethal treatment is assessed to have been successful.

The present invention also concerns a method of treating one or more plants which comprises applying a lethal treatment to the plant and testing the effectiveness of the lethal treatment.

The invention also concerns methods for validating a lethal treatment applied to a root or rhizome system of a plant.

The invention further concerns methods for optimising lethal treatments applied to a root or rhizome system of a plant and methods of obtaining an assessment of the effectiveness of a lethal treatment.

Background of the Invention

An unwanted infestation of plants can cause a number of problems such as choking slower growing or smaller plants, changing the ecology of a site and, causing structural damage to nearby buildings and infrastructure including paving and hardstanding as well as buried services such for example as sewerage, depending on the type of plant species which is growing out of control.

Any type of plant may be classed as a weed if it grows on a site where it is not wanted, including horticultural species. Such plants may be difficult to remove, especially if they are fast growing, spread easily and/or are resistant to weed killers or other treatments used in their removal. A number of invasive plants species are particularly hard to remove. Invasive plants are non-native species which can grow particularly rampant and choke native fauna and change the ecology of a site, in some cases over a short time period.

Japanese knotweed (*Fallopia japonica*) is a highly invasive plant species found in the UK and across Europe and North America, with an estimated frequency of one area of infestation every 10 km$^2$ in the UK. Native to East Asia, the growth of Japanese knotweed is controlled by natural pests and diseases in its natural habitat (see, e.g., Beerling, D., Bailey, J., & Conolly, A. (1994). Fallopia Japonica (Houtt.) Ronse Decraene. Journal of Ecology, 82(4), 959-979. doi:10.2307/2261459 and RPA (2020): International Approaches to Japanese Knotweed in the Context of Property Sales, Final Report for Defra, September 2020, Norwich, Norfolk, UK). However, once introduced into the UK and elsewhere in Europe in the mid-nineteenth century, Japanese knotweed spread rapidly owing to a lack of environmental pressures. Japanese knotweed is particularly prevalent along watercourses, railways and other transport routes and many urban areas, and especially brownfield and development sites. Owing to the highly invasive nature of this plant, it has a detrimental impact on the environment, structures (such as riverbanks) and buildings and hardstanding. It is therefore associated with significant economic cost, both in terms of the damage it causes and the cost of treating and removing an infestation.

Japanese knotweed grows quickly, growing up to 40 mm per day during late spring and early summer, to reach heights of over 2.1 m. Japanese knotweed is a herbaceous perennial which dies back to ground level in the winter months leaving the underground network of rhizomes lying dormant and only dead stems above ground. In spring, new growth and shoots grow from the rhizomes between the old, dead growth, to form a dense clump of canes (or stems) above ground. The rapid growth of Japanese knotweed means that it can quickly takeover a significant area, blocking light to slower growing plants beneath and suppressing the growth of other plants. This adversely affects the ecology of an area and can significantly alter a habitat (Global Invasive Species Database, 2020).

Owing to its prevalence along watercourses and railway lines, Japanese knotweed may cause structural damage to train tracks and erode the soil along waterways, increasing the risk of flooding. Marginal land adjacent to a train track or the like may act as a corridor, allowing Japanese knotweed to spread unnoticed.

Japanese knotweed shoots are capable of growing through any weaknesses in asphalt and concrete, resulting in significant damage to infrastructure, buildings and foundations. For this reason, insurers and lenders have strict requirements for the removal of Japanese knotweed from contaminated sites.

The treatment, removal and disposal of Japanese knotweed is governed by several laws. In the United Kingdom, for example, the Environmental Protection Act 1990 lists Japanese knotweed as "controlled waste" which must be disposed of safely according to the Environmental Protection Act (Duty of Care) Regulations 1991, and preferably following the Invasive Non-native Specialists Association (INNSA) UK Code of Practice for managing Japanese Knotweed. It is an offence to plant, or otherwise let grow in the wild, Japanese knotweed in the UK under Section 14(2) of the Wildlife and Countryside Act 1981. Compliance with such Regulations and/or the Code of Conduct is typically time consuming and expensive.

Removal of Japanese knotweed is challenging, especially for well-established, mature plants. It is highly resilient to treatment by foliar herbicide application and above ground mowing or combustion. Moreover, elimination of the plant must be very carefully managed to prevent re-infestation of an area. Japanese knotweed can extend over a wide area, with its rhizome network potentially reaching a depth of at least 2 m below ground, and potentially extending laterally to a distance of at least 7 m from the parent plant. Small pieces of rhizome of as little as 0.7g in fresh weight are capable of growing into a new plant. This adds to the complexity and cost of removing Japanese knotweed, and also the management of site excavated materials which might otherwise be re-usable.

Knotweed stores nutrients in its underground root system, enabling it to go dormant in the winter months. However, the plant is capable of growth many months later. Japanese knotweed rhizomes can persist for long periods of time in a viable but un-germinated or dormant state, particularly if the rhizome has undergone shock, such as during cold weather or following herbicide treatment. This means that a dormant rhizome can still cause re-infestation years later.

Other such plant species that may require lethal treatment to remove them from a contaminated site include other species of knotweed, such as, dwarf Japanese knotweed (*Fallopia japonica var. compacta*), Giant knotweed (*Fallopia sachalinensis*), Bohemian knotweed (*Fallopia x bohemica*), Himalayan knotweed (*Polygonum polystachyum*) and Railway yard knotweed (*Fallopia x conollyana*). Other plant species and varieties which may also be regarded as weeds and therefore candidates for lethal treatment are Rosa rugosa, rhododendron, buddleja, ferns, horsetails, brambles, nettles and bamboo, or any other perennial plant that is growing in a location where that is undesirable.

Known methods for treating plants to kill them and/or to remove them from a site include chemical (e.g. herbicide), mechanical (e.g. mowing, shading - for example with mulching sheets - , containment, - for example with geomembranes - , or excavation and removal), physical (e.g. by heating or electrocution) and biological (e.g. biocontrol agents) techniques.

The excavation and removal method (which is also known colloquially as "dig and dump") is a process in which ground is excavated and contaminated soil taken off-site and disposed of; for example at a licensed landfill site. Another method involves excavation and on-site lethal treatment. In this method, contaminated soil is removed and then screened to allow uncontaminated materials to be returned safely to the site and reduce volumes for off-site disposal. Alternatively, in some scenarios, infested areas may be contained (e.g. using geomembranes) or an infestation may be excavated and buried on site in specially designed containment systems.

Chemical methods of treating an infestation by an invasive plant species or weed include foliar application of a herbicide, whereby herbicide is applied to the leaves of the plant. However, care needs to be taken to avoid accidentally spraying other plants with the herbicide and caution used in applying this method near water. As an alternative, stem injection may overcome many of the problems associated with foliar spraying of herbicide as it involves a small dose of herbicide injected into the stem of the plant in a targeted approach (see, for example, WO 2005/004583 A1, GB 2,384,158 A).

Hannover Milieu- en Veiligheidstechniek BV (HMVT) (Ede, Netherlands) have disclosed thermal treatment and remediation of soil infested with Japanese knotweed by injecting hot air into the root-zone of the soil though a plurality of spaced injectors.

Given its ability to regrow from small fragments of rhizome, killing and/or complete removal of Japanese knotweed rhizomes is crucial in eradicating it from a contaminated site. However, it is extremely difficult to determine whether or not a lethal treatment applied to a plant, and in particular its rhizomes, has been successful in killing it. This also holds true for many different weeds having root or rhizome systems. Distinguishing between dead, weakened and viable yet dormant plants is particularly difficult. For example, once a lethal treatment has been applied, a plant may be shocked into dormancy and appear to be dead, but is still in a viable state and able to regrow. This could result in plants which are believed to be dead, but are in fact viable in a dormant state, being left unsuccessfully treated, leading to a site being re-colonised. In turn, this adds to the cost of eradicating plants from a site, both in terms of time and money, as multiple treatments will often be applied to plants to increase the degree of certainty that they are dead such that re-infestation cannot occur. Although the problem of identifying whether or not a plant is dead is particularly crucial for Japanese knotweed and other knotweed species, given their highly invasive nature, it will be appreciated that it is also advantageous to be able to determine accurately whether or not any kind of weed or plant that is growing in an undesirable location is dead or alive.

Owing to the fact that it is difficult to determine whether or not plants, and in particular the rhizomes or wider root systems of such plants, are dead or merely dormant, reliable viability testing of rhizomes / roots would be highly advantageous in informing the management and clearance of infestations and, consequently, reducing the cost of insuring against the failure of treatments. The ability to determine accurately whether or not a lethal treatment has successfully killed a plant would alleviate the need for retreatment in cases where it is not needed. However, conventional viability testing is based on observations of re-germination over a period of several weeks; for example of plant material in compost. This process is time consuming and unreliable.

Known viability testing methods can determine whether a plant is alive, but cannot conclude with a high degree of accuracy that a plant is dead. There is therefore a need for rapid and highly accurate and reliable testing to determine whether or not a treated plant is really dead following a lethal treatment, or if the plant is still alive and needs to be retreated.

The present invention seeks to mitigate one or more of the above-mentioned problems. Alternatively or additionally, the present invention seeks to provide an improved method of determining the effectiveness of a lethal treatment applied to plant species; for example Japanese Knotweed.

Summary of the Invention

In a first aspect of the present invention therefore there is provided a method of determining the effectiveness of a lethal treatment applied to a plant or a group of two or more plants of the same plant species or variety; the method comprising assessing whether the treated plant or group of plants is dead on the basis of a comparative electrolyte leakage value ($CEL_{sample}$) of one or more root or rhizome samples taken from the treated plant or group of plants; wherein $CEL_{sample}$ is obtained by:

a) measuring the electrolyte leakage of a root or rhizome sample ($EL_{sample}$);
b) thereafter, exposing the sample to cell barrier disrupting conditions;
c) measuring the electrolyte leakage of the sample ($EL_{total}$) again, after the sample has been exposed to the cell barrier disrupting conditions; and
d) calculating $CEL_{sample}$ as:

$$CEL_{sample} = 100 \times \left( \frac{EL_{sample}}{EL_{total}} \right).$$

It may be that the $CEL_{sample}$ value is represented as a numerical range between 0 to 1 as an alternative to representing $CEL_{sample}$ as a percentage value. Thus, it may be that $CEL_{sample}$ is determined as the value of $EL_{sample}/EL_{total}$. The skilled person will understand that a $CEL_{sample}$ value expressed as a value between 0 to 1 is equivalent to, and interchangeable with the same $CEL_{sample}$ value expressed as a percentage. For example, $CEL_{sample}$ values, including mean $CEL_{sample}$ values as described herein, may be expressed either as a percentage or value between 0 and 1.

In some embodiments, the method may comprise assessing the status of the or each sample individually on the basis of its $CEL_{sample}$ value. Suitably, the method may comprise comparing the $CEL_{sample}$ value with a first critical value (C1) and indicating the sample to be dead when $CEL_{sample}$ is equal to or greater than C1.

In some embodiments, the method may further comprise comparing the $CEL_{sample}$ value with a second critical value (C2) and indicating the sample to be alive when $CEL_{sample}$ is less than C2. It will be understood that C2 is less than or equal to C1.

The method may comprise indicating the status of the sample to be indeterminate when $CEL_{sample}$ is equal to or greater than C2 and less than C1.

In some embodiments, $CEL_{sample}$ values for a plurality of samples taken from the plant or group of plants may be assessed. Suitably, a plurality of samples (e.g. 2-10 samples, typically 2-3 samples) may be taken from a single segment of the root or rhizome. In some embodiments a plurality of segments taken from the plant or group of plants may be tested. Suitably, the $CEL_{sample}$ values of one or more samples from each segment may be measured.

Alternatively, the method of the invention may comprise calculating individual $CEL_{sample}$ values for a plurality of samples of the plant or group of plants, deriving a mean $CEL_{sample}$ value from the individual $CEL_{sample}$ values, and assessing whether the treated plant or group of plants is dead on the basis of the mean $CEL_{sample}$ value.

Again, individual $CEL_{sample}$ values for a plurality of samples taken from one or more segments of the plant or group of plants may be measured. Suitably, a plurality of samples may be taken from multiple roots and/or rhizomes collected from a plant or group of plants. For example, at least one sample may be obtained per root and/or rhizome collected, wherein two or more root and/or rhizomes are collected per plant or group of plants. Suitably, a plurality of samples (e.g. 2-10 samples, typically 2-3 samples) may be taken from a single segment of the root or rhizome. The mean $CEL_{sample}$ value may be calculated from the individual $CEL_{sample}$ values for the samples from the one or more segments.

Preferably, the plurality of samples of the plant or group of plants are obtained from roots and/or rhizomes collected from a site where the plant or group of plants are located, wherein the site is divided into multiple unit areas or unit volumes, and wherein the roots and/or rhizomes are collected from a proportion of the total unit areas or unit volumes. Thus, only a proportion of the total number of samples that could be retrieved from a site are actually analysed. It may be that whole roots and/or rhizomes are collected from a site. Additionally or alternatively, it may be that sections of roots and/or rhizomes are collected from a site. It may be that an entire root and/or rhizome is obtained from a site and is then cut into sections. The collected roots and/or rhizomes or sections thereof may be analysed by electrolyte leakage testing methods disclosed herein. Advantageously, only a proportion of samples available from a site are selected and analysed by the methods disclosed herein so as to reduce the number of samples that are to be processed. A proportion of samples may be selected by any of the sampling methods disclosed herein, for example through the use of excavation units and/or trial pits. Accordingly, the methods provided herein may comprise collecting a plurality of root and/or rhizomes or sections thereof, from a site. The method may further comprise obtaining samples from at least a proportion of the plurality of root and/or rhizomes collected and analysing the samples by an electrolyte leakage testing methodology disclosed herein.

Preferably, only a proportion of roots or rhizomes or sections thereof, collected from a site are analysed according to methods disclosed herein to determine the $CEL_{sample}$ value. When the samples are obtained from rhizomes, it may be that the collected rhizomes or sections thereof that are identified as rotten by visual inspection (and are therefore non-viable or dead) are excluded from electrolyte leakage testing and the $CEL_{sample}$ value of those rhizomes is not determined. It may be unnecessary to analyse by electrolyte leakage testing rhizomes that are assessed as rotten by visual inspection, as it can be fairly concluded by visual inspection alone that these sample are dead or non-viable. It may be that the total number of rhizomes or sections thereof collected from a site that are assessed to be rotten by visual inspection is determined and combined with the results of the electrolyte leakage testing of non-rotten rhizomes to draw a conclusion as to the overall viability of a plant population.

Thus, the method of the invention may comprise comparing the mean $CEL_{sample}$ with a first critical value (C1) and indicating the plant or group of plants to be dead when mean $CEL_{sample}$ is equal to or greater than C1.

Preferably, the method comprises determining whether the mean $CEL_{sample}$ value is indicative that an Acceptable Outcome has been reached, and/or determining whether the mean $CEL_{sample}$ value is indicative that an Unacceptable Outcome has been reached. An Acceptable Outcome is such that it is likely that fewer than v(a)% of plant material, such as roots and/or rhizomes, of the plant or group of plants to which the lethal treatment has been applied have a $CEL_{sample}$ value of less than C1. An Outcome (Acceptable or Unacceptable) is likely if the probability of achieving that outcome is at least 80%, at least 90%, at least 95% or at least 99.5%. An Unacceptable Outcome is such that it is likely that more than v(u)% of plant material, such as roots and/or rhizomes, of the plant or group of plants to which the lethal treatment has been applied have a $CEL_{sample}$ value of less than C1. Preferably, v(a)% is <0.01% and/or v(u)% is > 1.0%. When the mean $CEL_{sample}$ value is indicative that an Unacceptable Outcome has been reached, a second lethal treatment may be applied to the plant or groups of plants to which a lethal treatment had already been applied. Preferably, the mean $CEL_{sample}$ value is obtained by carrying out electrolyte leakage testing on a proportion of plant material (such as roots and/or rhizomes) available from a plant or group of plants, for example a group of plants present at a site, according to methods presented herein and the Unacceptable Outcome and/or Acceptable Outcome are used to assess whether the mean $CEL_{sample}$ value obtained from a proportion of available plant material is indicative of the status of the entire population.

The mean $CEL_{sample}$ value may indicate that neither an Acceptable Outcome or an Unacceptable Outcome has been reached such that more than v(a)% of plant material, such as roots or rhizomes, of the plant or group of plants to which the lethal treatment has been applied are likely to have a $CEL_{sample}$ value of less than C1, but less than v(u)% of plant material, such as root or rhizomes, of the plant or group of plants to which the lethal treatment has been applied are likely to have a mean $CEL_{sample}$ value of less than C1. In such a scenario, it may be that further samples of plant material are obtained from the plants or group of plants to which a lethal treatment has been applied and tested by electrolyte leakage testing as disclosed herein. The mean $CEL_{sample}$ value may be recalculated by incorporating the $CEL_{sample}$ values of the further samples in addition to the $CEL_{sample}$ values previously determined. The method may further comprise determining whether the recalculated mean $CEL_{sample}$ value is indicative that an Acceptable Outcome has been reached, and/or determining whether the recalculated mean $CEL_{sample}$ value is indicative that an Unacceptable Outcome has been reached. When the mean $CEL_{sample}$ value is indicative that neither an Unacceptable Outcome or an Acceptable Outcome has been reached, a second lethal treatment may be applied to the plant or groups of plants to which a lethal treatment had already been applied.Further, the method may comprise comparing the mean $CEL_{sample}$ value with a second critical value (C2) and indicating the plant or group of plants to be alive when mean $CEL_{sample}$ is less than C2.

Again, it will be understood that C2 is less than or equal to C1. When C2 is less than C1, the method may comprise indicating the status of the plant or group of plants to be indeterminate when mean $CEL_{sample}$ is equal to or greater than C2 and less than C1, in which case the plant or group of plants may be retested using a method of the invention or a different, complementary method, as described in more detail below.

The critical values C1 and, optionally, C2 may allow the effectiveness of a lethal treatment to be determined in accordance with some embodiments of the invention with at least 95% confidence.

As used herein, the term "plant" relates to any plant which receives a lethal treatment which is intended to kill the plant. The plant can be a weed, so defined as a plant that is growing in an undesirable location, typically in an uncontrolled manner. Weeds include perennial species, native and non-native plant species, such as invasive plants.

As used herein, an "invasive plant species" means an alien species which shows a tendency to spread out of control. Invasive plant species include, but are not limited to Japanese knotweed.

Other plant species might not be considered to be invasive if they are native to the area where they are growing, but may nonetheless cause problems if they are growing on sites where they are not wanted. Such plant species may be described as weeds. It will be understood that herein a reference to "plant" or "plant species" refers to any plant species which may be growing in a location where it is not wanted and thus is treated to manage, control or eliminate such a plant from a site.

As used herein, a "rhizome" means a somewhat elongated, usually horizontal subterranean plant stem that is often thickened by deposits of reserve food material, produces shoots above and roots below, and is distinguished from a true root in possessing buds, nodes, and usually scale-like leaves (Miriam Webster, accessed 18 September 2020). The methods of the inventions may be suitable for determining the effectiveness of a lethal treatment applied to a plant having rhizomes. In particular, the methods may be applied to a plant having lignified rhizomes. A "lignified rhizome" means a rhizome which has become woody through the formation and deposit of lignin in its cell walls. The methods of the present invention may also be applied to root systems of a plant. Japanese knotweed is a plant having lignified rhizomes.

As used herein, the term "group of two or more plants" may refer to two or more individual plants found within a close proximity to one another; for example less than 10-15 m apart. In can often be difficult to tell whether parts of a plant, especially those parts which grow underground, are from the same or different individual plants. For example, a group of two or more plants may form a cluster or clump of plants found within close proximity to one another, each plant sharing substantially the same genetic material. Plants which are clones from a parent plant are considered to be a group of two or more plants. Thus in some embodiments of the invention, the method may comprise testing one or more samples from parts of plants (e.g. roots or rhizomes) found within the same area of a site, e.g. within about 10-15 m, and preferably within 1-5 m.

It will be understood that for testing the efficacy of a lethal treatment that is applied to a large area site, it may be desirable to test a plurality of samples which are harvested from a plurality of spaced locations across the site. Various standards are available to those skilled in the art for sampling a site for chemical analytes, e.g. ISO 15176:2019 *Guidance on characterization of excavated soil and other materials intended for re-use* (https://www.iso.org/standard/70771.html), BS 10175:2011+A2:2017 *Investigation of potentially contaminated sites. Code of practice* (https://shop.bsigroup.com/ProductDetail?pid=000000000030362551) and standards which are linked to these. Those skilled in the art may adapt standards of this kind for use in root or rhizome sampling at a site. In some embodiments, multiple samples may be taken in the vicinity of a given location in accordance with a standard for sampling until a rhizome or root is found.

Suitably, one or more samples may be taken from one or more plants at each location, as described above. The different locations may be spaced apart by about 1-20 m, preferably about 5-15 m, e.g. about 10 m. The samples may also be taken from different depths. The different depths may be spaced apart by a depth of about 0.01-1 m, preferably about 0.25-0.75 m e.g. about 0.5 m. In some embodiments, one or more samples of roots or rhizomes may be taken from one location per unit volume of soil. Suitably, the unit volume may be 50-150 $m^3$, e.g. about 100 $m^3$. Suitably, one or more segments of roots or rhizomes may be harvested from each location; for example 2-5 segments per unit volume of soil, e.g. 3 segments. When testing the segments, the $CEL_{sample}$ values of one or more samples from each segment may be measured, as described above.

The methods of the invention involve determination of the electrolyte leakage of a sample before and after the sample has been subjected to cell barrier disrupting conditions. Such determination of electrolyte leakage is referred to herein as electrolyte leakage testing (ELT). Preferably, the sample is thoroughly cleaned before ELT, for example by removal of any soil on the surface of the sample. This may increase the accuracy of the ELT method as contaminates may include salts which alter the $CEL_{sample}$ value obtained by ELT.

As used herein, "electrolyte leakage" is defined as the conductivity of a sample in water which results from diffusion of electrolytes from inside the cell into the water surrounding the sample. The water is initially substantially non-conducting. For example, the water is of sufficient purity to make it possible to detect the release of cell electrolyte into the water. Preferably, the water is substantially free of electrolytes, for example the water is deionised water. The greater the extent of cell damage in the sample, the higher the electrolyte leakage. Electrolyte leakage may be measured according to methods described herein.

As used herein, the term "cell barrier" refers to the cell wall and/or cell membrane. In other words, the cell barrier is the layer(s) of the cell that controls passage of electrolytes from inside to outside of the cell.

The term "cell barrier disrupting conditions" is used herein to mean the conditions to which a rhizome or root sample from a plant is subjected as part of the testing methods in accordance with the present invention. A sample of a rhizome or root will normally be killed under such cell barrier disrupting conditions, which will typically be more severe than the treatment to which an entire plant could be subjected in practice. For example, cell barrier disrupting conditions may include heat treatment or exposure to ionising (e.g. gamma) or microwave radiation or sonication or a chemical agent. The precise conditions used are not essential provided that the conditions cause disruption of the cell barrier such that electrolytes will leak through the cell barrier, for example into a surrounding solution.

The methods according to the first aspect of the present invention allow a sample of root or rhizome obtained from one or more plants to be tested after the one or a group of two or more plants have received a lethal treatment, in order to assess whether the one or more plants have been successfully killed by the lethal treatment.

By "lethal treatment" or simply "treatment" herein is meant a procedure or method comprising one or more steps which are calculated to kill a plant, typically in the environment, at or near the site where it is growing. Such treatments are used to manage, control or eliminate plants from a site. A lethal treatment may be mechanical, chemical, physical and/or biological. Previously, multiple herbicide treatments have typically been applied to plants to ensure that all plants on a site are killed. However, this can be time consuming and costly. The methods according to the present invention may be used to determine whether a treatment of one or more plants has been successful with about 95% confidence or more, based on the $CEL_{sample}$ or mean $CEL_{sample}$ value of one or more samples obtained from a treated plant or group of plants, and therefore means that unnecessary further treatments may be avoided.

The lethal treatment may be applied to any part of a plant, and preferably, although not exclusively, is applied to the root or rhizome system of the plant. If a site contains more than one plant, then the lethal treatment may be applied to each of the plants, for example, the entire root or rhizome system present at the site.

It will be understood that when more than one plant has been treated, for example several plants have been treated on a single site, the sample or samples taken for analysis do not need to be assigned or identified as belonging to a specific plant that has been treated.

For example, if several plants are treated, then a sample obtained from only one plant may be sufficient to determine whether or not the treatment has been effective for all the plants. It is not necessary that such as sample is attributed to a specific plant out of the several plants that were treated. If a site is disrupted after the plants were treated, for example by digging or ploughing, then it may in any event be impossible to assign a particular sample taken from a root or rhizome to a particular plant that was treated. What is important is that a representative sample or plurality of samples (e.g. in the case of a large area site) is taken from the one or more plants that have been treated.

The skilled person will readily be able to determine the value C1 and/or C2 for a given species and/or a given situation according to the procedure described herein.

Suitably, C1 may be determined by reference to $CEL_{sample}$ values that have been obtained from a plurality of known dead reference samples taken from rhizomes or roots of the same invasive plant species or variety as the treated plant or plants. The critical value C1 may be selected as the $CEL_{sample}$ value that identifies the dead reference samples with at least about 95% accuracy.

Thus, in some embodiments, the critical value C1 may be calculated as mean $CEL_{sample}$ -2SD of the dead reference samples.

The known dead reference samples may comprise a plurality of samples originating from each one of a plurality of different sites. The critical value C1 may be calculated as a mean site mean $CEL_{sample}$ -2SD of mean $CEL_{sample}$ values of the dead reference samples from the different sites.

Meanwhile, C2 may be determined by reference to $CEL_{sample}$ values that have been obtained from a plurality of known live reference samples taken from rhizomes or roots of the same invasive plant species or variety as the treated plant or plants. The critical value C2 may be selected as the $CEL_{sample}$ value that identifies the live reference samples with at least about 95% accuracy.

Thus, in some embodiments, the critical value C2 may be calculated as mean $CEL_{sample}$ +2SD of the live reference samples.

As with the dead reference samples, the known live reference samples comprise a plurality of samples originating from each one of a plurality of different sites, and the critical value C2 is calculated as a mean site mean $CEL_{sample}$ +2SD of mean $CEL_{sample}$ values of the live reference samples from the different sites.

In some embodiments, as described in more detail below, the plant or plants from which the reference samples are obtained may be grown under the same conditions as the plant to be treated is growing. Further, or alternatively, the reference samples may be obtained from one or more plants at a similar time of year as the lethal treatment is applied to the plant to be treated.

In some embodiments, C1 may be a value between about 45% and about 75%; preferably between about 45% and about 60%; more preferably between about 47% and about 52%. In some embodiments, C1 may be about 48.5%. In some embodiments, C1 may be between about 50% and about 60%. In some embodiments, C1 may be about 50%, or preferably about 56%. When represented as values between 0 and 1, C1 may therefore be between about 0.5 and 0.6, more preferably about 0.56. As disclosed herein, C1 may be chosen in dependence of the distribution of $CEL_{sample}$ values obtained from live and dead reference samples. A critical value within one of these ranges or of this order may be especially suitable when the plant species is Japanese knotweed, and may allow differentiation of live and dead samples obtained from treated plants to more than about 95% accuracy. Without wishing to be bound by theory, different plant varieties and species are expected to have a C1 value falling within these ranges, owing to the similarities between the different varieties and species of plants of the cell barrier physiology, which controls electrolyte leakage from the cell. Generally, in accordance with the present invention, C1 is a value against which the $CEL_{sample}$ of a sample or mean $CEL_{sample}$ of a plurality of samples obtained from a plant or a group of plants is compared in order to assess whether a treated plant is dead with a confidence of > about 95% accuracy.

In some embodiments, C1 may be a value between about 55% and about 75%; for example about 57.5%. Such a value of C1 may increase the accuracy of the method even further so that a treated plant may be indicated to be dead with at least 95% accuracy; preferably at least 97% accuracy; and more preferably with an accuracy equal to or greater than 99%.

In some embodiments, C2 may be a value which is less that C1 and between about 30% and about 60%; preferably between about 32% and about 45%; for example about 37.5%. Generally, in accordance with the present invention, C2 is a value against which the $CEL_{sample}$ of a sample or mean $CEL_{sample}$ of a plurality of samples obtained from a plant or a group of plants is compared in order to assess whether a treated plant is alive with a confidence of > about 95% accuracy.

In some embodiments, C2 may be a value between about 30% and about 40%; for example about 32%. Such a value of C2 may increase the accuracy of the method even further so that a treated plant may be indicated to be alive with at least 95% accuracy; preferably at least 97% accuracy; and more preferably with an accuracy equal to or greater than 99%.

Methods of the invention which involve comparing $CEL_{sample}$ of one or more samples or mean $CEL_{sample}$ of a plurality of samples from a plant or a group of two or more treated plants against both C1 and C2 may thus allow a plant or group of plants to be identified as having been successfully treated ($CEL_{sample}$ or mean $CEL_{sample}$ is equal to or greater than C1), unsuccessfully treated and therefore requiring further lethal treatment ($CEL_{sample}$ or mean $CEL_{sample}$ is less than C2), or an indeterminate result ($CEL_{sample}$ or mean $CEL_{sample}$ is equal to or greater than C2, but less than C1). Where $CEL_{sample}$ of a plurality of samples of a plant or group of plants are measured, the assessment of the plant's or group of plants' status may be assessed using statistical methods well known to those skilled in the art, which need not be described herein.

In the case of an indeterminate result, a sample or plurality of samples may be retested in accordance with a method of the present invention and/or one or more different methods, to determine the status of the sample(s) and thus whether the plant or plants have been successfully treated. It will be understood that testing a plurality of samples and assessing their respective individual $CEL_{sample}$ values (for example using standard statistical methods) or assessing their mean $CEL_{sample}$ value will generally produce a more accurate result, since it allows false positive or negative results to be identified and discarded and/or reduces the dependence of the overall assessment on a single sample.

Optionally, therefore, when $CEL_{sample}$ or mean $CEL_{sample}$ is less than C1 and equal to or greater than C2, a complementary method may be used to determine the status of the sample. Suitably, the complementary method may be a propagation testing method. Such a propagation testing method relies on observing biological growth in a sample that is not dead. A sample that is not dead may grow roots once subjected to propagation testing.

In some embodiments, such a propagation testing method may comprise incubating a section of rhizome in a suitable growth medium, such as soil, perlite or agar gel. Suitably, a thin section of rhizome may be incubated on agar gel for about 6 days or more. If root hairs develop on the rhizome sample, then the sample is identified as alive. However, if no root hairs develop then the sample is identified as dead.

The methods of the invention may be performed off-site (for instance under laboratory conditions) or on-site (for instance in the field, e.g. at the same location as where the plant is treated). Quantitative results are obtained which may be used to indicate whether or not a lethal treatment applied to a plant species has been successful.

As discussed above, Japanese knotweed, and other plant species, may be particularly difficult to identify as dormant, weakened or dead, especially after the plant has been treated; for example with a lethal treatment. It is important that the state of the plant or group of plants is identified correctly, because leaving a plant which is believed to be dead, but is in fact still alive, on-site may lead to re-colonisation later; for example when the plant emerges from a dormant state. The present method seeks to identify whether a plant is dead following lethal treatment with a confidence of at least 95%.

Unlike previous methods that have been applied to fine root systems of non-invasive species of trees which are grown under nursery conditions to check their viability for planting in managed forests, the present methods are applied to roots and rhizomes of invasive plant species and other weeds, to assess whether they are dead with a high degree of confidence. The accuracy of the methods according to the present invention allows a user to be reasonably confident that a site is completely clear of the plant following a lethal treatment and reduces the level of risk for insurers offering insurance-backed guarantees of the effectiveness of the treatment and lenders taking a security interest in a property on or adjacent to a treated site.

The reduction in unnecessary repeat treatments may also reduce the amount of herbicide which may be used in treating plants and may therefore reduce the environmental impact. Likewise, if any other or alternative treatments, such as biological, chemical or physical treatments are used, a reduction in the number of times this treatment is to be repeated or the amount of time a plant is to be treated for will limit the environmental impact, as well as the cost associated with such treatments.

The methods for determining the effectiveness of a lethal treatment according to the present invention are typically rapid and may give results in as little as about 1.5 days.

Selecting C1 and/or C2 by reference to the $CEL_{sample}$ values of a plurality of dead and/or live reference samples taken from rhizomes or roots of the invasive plant species or variety to be treated, as described above, may allow the reliability of the methods of the present invention to be optimised by taking into account any variations in C1 and/or C2 value for different plant species and varieties. Although use of a fixed value for C1 and/or C2 may be sufficient to determine the effectiveness of a lethal treatment with $\geq$ 95% accuracy, use of C1 and/or C2 determined from a plurality of reference samples may increase the reliability of the methods.

For instance, in some embodiments, the $CEL_{sample}$ values of live and dead samples of roots or rhizomes obtained from plants may vary between different samples of the same plant species, depending on the time of year the plants are tested or the conditions under which the plants have grown. Suitably, therefore, the plant or plants from which the reference samples are obtained should have been grown under the same growing conditions as the plant to be treated. Thus, in some embodiments, the critical values C1 and/or C2 may suitably be derived by reference to measured $CEL_{sample}$ values for a plurality of known dead and/or live reference samples taken from rhizomes or roots of one or more plants of the same plant species or variety as the plant or plants to be treated, which have been grown under the same conditions as the plant or group of plants to be treated. In some embodiments, the reference samples may be cut from a plant or plants at the same time of year as the lethal treatment is to be applied to the plant or group of plants to be treated.

Generally, the growing conditions of the plants from which the reference samples are taken include, but are not limited to, soil conditions, climate and altitude.

The time of year at which reference samples are obtained includes, but is not limited to, spring, summer, autumn or winter.

In some embodiments, the reference samples may be taken from one or more plants which have grown at the same or a similar geographical location as the plant or plants to be treated.

By a "site" herein is meant a continuous area of land in which the plant is growing and distributed. Several plants of the same species may grow and be distributed across a single site. It may be that a particular plant species, for example Japanese knotweed, only populate a percentage of the surface area or volume of the site, for example less than 70%, less than 60% or less than 50% of the surface area or volume of site. Thus, there may be areas of land of the site which are devoid of signs of the particular plant species, such as leaf or stem material, or roots or rhizomes. It may be that there are localised populations of the plant species at different areas or locations of the site, the localised populations being separated by areas without any signs of the plant material. Such localised populations may be termed a "sub-population", whereas the entire population of plants at a site may be termed the "whole population". It may be that the methods described herein are applied to a representative sample of the whole population, or the methods described herein may be applied to a representative sample of each sub-population. A site may be characterised by its geographical location, and thus different sites may have different geographical locations. A site may additionally or alternatively be characterised by its growing conditions; for example, soil conditions at the site. Thus, areas having different soil conditions may be identified as different sites even if they share the same geographical location.

Suitably, the reference samples may be obtained from plants grown at least at two different sites. In some embodiments, an even greater degree of accuracy may be achieved by deriving C1 and/or C2 from $CEL_{sample}$ values of reference samples obtained from more than two sites; for example three sites, five sites, ten sites, twenty sites or thirty sites or more. As described above, for example, C1 and/or C2 may be based on mean site mean $CEL_{sample}$ values.

Dead reference samples may be prepared, for example, by heating a sample of root or rhizome at about 50 °C for about 2 hours. The skilled person will understand that any method which kills the sample while leaving the sample intact for ELT may be suitable.

Comparing the $CEL_{sample}$ value of a sample of root or rhizome from a treated plant or group of plants or the mean $CEL_{sample}$ value of a plurality of such samples to values for C1 and/or C2 based on reference samples as described above may allow samples obtained from a wide range of different plant species to be tested using the methods of the present invention, as well as samples taken at different times of year or from different environments.

In some embodiments, the cell barrier disrupting conditions of step b) of the present methods may comprise exposing the sample to at least one of sonication, ionising radiation, heat treatment, microwave radiation, radiofrequency radiation, physical attrition and/or a chemical agent, including altering the pH of an aqueous medium containing the sample.

In some embodiments, the treatment may comprise a heat treatment in which the sample is heated to about 70 °C or more, preferably about 80 °C or more, or more preferably about 90 °C or more. The sample may be heated at this temperature for at least a certain minimal length of time; for example at least 5 minutes, at least 10 minutes or preferably at least 20 minutes. Most preferably, when a heat treatment is applied, the sample is heated to about 90 °C for at least 20 minutes.

The cell barrier disrupting conditions are those that are suitable for disrupting the cell barrier of the root or rhizome sample, thus allowing electrolytes to leak from the sample. When the sample is submerged in deionised water, the electrolytes will leak into the water and the conductivity of the solution can be measured to indicate whether the sample was alive or dead following a treatment. Advantageously, the use of a heat treatment for this step avoids the need for long exposure times or particularly high temperatures, making it easy and quick to perform.

Alternatively or additionally, the cell barrier disrupting conditions may be one or more of ionising (e.g. gamma) radiation, microwave radiation, or sonication. It will be understood that any cell barrier disrupting conditions that result in the cell barrier of the sample being disrupted so that electrolyte leaks out of the sample are suitable for use in step b).

Optionally, the sample of root or rhizome may be submerged in water before step a). Preferably the water is substantially free of electrolytes; for example the water may suitably be deionised water. Suitably, the sample may be stored in the dark before step a). Additionally or alternatively, the sample may be stored at room temperature before step a). The sample may be stored for at least 12-16 hours, for example overnight, before step a). In some embodiments, the sample may be stored in the deionised water for at least about 16 hours before measuring the electrolyte leakage of the sample ($EL_{sample}$) in step a). Advantageously, one or more of these procedures may ensure that the sample has time to stabilise and the conductivity of the solution to equilibrate.

Suitably, the storage of the sample may comprise sealing the sample in a container as soon as the sample has been cut from the rhizome or root of the plant. Optionally, the sample may be cut from the root or rhizome at the site from which the root or rhizome was obtained. Preferably, the container may be a sealed vial or bottle. Preferably, the container may be thermally insulated to keep the sample at a constant temperature. Preferably, the sample may be stored at a temperature of between 18 °C and 25 °C. The sample may be stored during transit from the site where the sample was prepared to a site where ELT will be carried out. For example, the sample may be stored during transit for 12-16 hours. Advantageously, this may ensure that the sample has time to stabilise and the conductivity of the solution to equilibrate and ELT testing can be carried out as soon as the sample arrives at the site where ELT is to be carried out.

Suitably, the sample analysed in steps (a-d) may comprise a slice of the root or rhizome. The slice may have a thickness of between about 1 mm and about 3 mm. Suitably, the thickness may be about 2 mm. The sample may be obtained from a segment of rhizome, preferably a lignified rhizome. The rhizome segment may have a diameter of between about 10 mm and about 30 mm. Ideally, the segment should be free of damage or blemishes. This may facilitate the provision of accurate electrolyte readings of the sample. Suitably, the sample should be analysed as soon as possible after harvesting the root or rhizome segment, with a view to ensuring the accuracy of the methods of the invention. In some embodiments, the sample may be analysed within about 72 hours of being harvested, and preferably within about 48 hours. However, provided it is kept moist and aerobic, a root or rhizome segment should normally stay viable for a long time.

For complementary testing based on regrowth, such as propagation testing as described above, a sample of rhizome must contain a node.

In some embodiments, one or more segments of the root or rhizome which are harvested from the plant or group of plants may be stored for up to 48 hours before testing.

Suitably, the one more segments should be stored in a breathable container, such for example as a bag formed of a suitably breathable material and/or provided with one or more openings to allow the bag to breathe. Segments and samples must not be frozen prior to testing, which might kill the rhizome. Segments from different plants or groups of plants should preferably be stored in separate containers. Suitably, rhizome segments may be at least about 10-15 cm long.

Optionally, the plant may be a species selected from Japanese knotweed (Fallopia japonica), Dwarf Japanese knotweed (Fallopia japonica var. compacta), Giant knotweed (Fallopia sachalinensis), Bohemian knotweed (Fallopia x bohemica), Himalayan knotweed (Polygonum polystachyum) and Railway yard knotweed (Fallopia x conollyana). Other plant species that can be tested include Rosa rugosa, rhododendron, buddleja, ferns, horsetails, brambles, nettles and bamboo and any other perennial plant that is growing in a location where that is unwanted. Most particularly, in accordance with the invention, the plant may be of the species Japanese knotweed (Fallopia japonica).

By way of example, when the plant species is Japanese knotweed, C1 may be about 48.5%. In some embodiments, C1 may be about 57.5%, more preferably, C1 may be about 56%. C2 may be about 37.5%. It has been found that with these values of C1 and C2 an assessment of the success of a lethal treatment applied to Japanese knotweed can be made with a high degree of confidence.

Optionally, the methods of the invention may further comprise assessing the $CEL_{sample}$ of at least one untreated sample of root or rhizome of the plant or group of plants or mean $CEL_{sample}$ of a plurality of samples of the plant or plants before a lethal treatment is applied; wherein $CEL_{sample}$ or mean $CEL_{sample}$ of the untreated sample is assessed against C1 and/or C2 as described above to ascertain the status of the plant before treatment. In this way it may be determined if in fact the one or more plants need to be treated. For example, it can be determined if the one or more plants are already dead before treatment. If the one or more plants are found to be already dead, then the method steps do not progress past this initial testing phase, as treatment of the one or more plants is not needed. If, however, the one or more plants are found to be still alive or if their status is indeterminate, then the method is continued and a lethal treatment is applied to the one or more plants, either by the same party performing ELT or a third party, and then ELT may be used according to the invention to determine the effectiveness of the treatment.

In some embodiments, methods of the present invention may further comprise subjecting one or more plants of the kind described herein having a root or rhizome system to a lethal treatment prior to determining the effectiveness of the treatment.

Thus, in a second aspect of the invention, there is provided a method for the the treatment of one or more plants, the method comprising:

i) applying a lethal treatment to one or more plants or groups of plants; and
ii) thereafter determining the effectiveness of the treatment in accordance with a method of the invention, as described herein.

Plants must be treated in order to kill them. Once killed, the plant can remain *in situ* to biodegrade or it may be removed from the site. Determining the effectiveness of the lethal treatment in killing the plant, according to the methods of the invention, may avoid the need for further, unnecessary treatments. This in turn may reduce the cost and time involved in treating an infestation of an invasive plant and minimise the environmental impact of treatment.

Optionally, the lethal treatment may be applied to the root or rhizome system of the one or more plants. In some embodiments, however, a stem or leaf treatment may be employed.

Suitably, the lethal treatment may comprise one or more of heat treatment, herbicide treatment, treatment with a chemical agent, electrical treatment, pH treatment and/or a stem injection treatment.

Heat treatments, herbicide treatments and stem injection treatments are known to be effective treatments for killing invasive plant species, such for example as Japanese knotweed. Such methods are disclosed in WO 2005/004583 A1 and GB 2,384,158 A, for example, the contents of which are incorporated herein by reference.

Stem injection treatments may be particularly advantageous because they may allow targeted injection of a herbicide into the stem of the plant. This may avoid contamination of neighbouring plants and minimise the environmental impact. Stem injection may be particularly suitable for use near watercourses.

Suitably, the lethal treatment may comprise a heat treatment, such as a steam treatment or hot air treatment. For example, a heat treatment may be applied to the soil surrounding the invasive species to a depth of at least 1m, preferably to a depth of at least about 2 m. The depth to which the soil is treated may be dependent, at least in part, on the level of the water table, as the treatment should not be applied to a depth greater than that of the water table.

The treatment may be applied to the soil *in situ* or *ex situ,* in the case of excavated soils.

Preferably, the heat treatment is heat treatment, such as a steam treatment or, more preferably, a hot air treatment.

Steam treatments may be particularly effective for treating soil to significant depths in order to kill an entire plant root or rhizome system. The rhizome systems of Japanese knotweed can extend to depths of about 2 m and so steam treatments which can be applied to this depth may be used to avoid excavation of the soil. If such steam treatments cannot be used at a particular site, then the soil may be excavated to a depth of about 2 m and placed in containers or enclosures which are adapted for steam treatment of soil placed within. Following steam treatment, the soil may be placed back on site, once it has been determined that the treatment has been successful in killing the plant.

It will be appreciated that treatment of one or more plants in situ on a site or ex situ taken from a site may take several days, typically one or more weeks.

In some embodiments of the invention in which a lethal treatment is applied to one or more plants, one or more reference portions of the same species as the plant or plants to be treated may be buried in soil with the plant or plants to be treated. The reference portions are suitably of known viability. For example, the reference portions may be known to be alive prior to application of the lethal treatment. The viability of the reference portions may be ascertained in any suitable manner which is known to those skilled in the art. For instance, the one of more reference portions may be ascertained to be alive using one or more of the methods described herein. Suitably, the reference portions should be used (e.g. buried) within about 72 hours of viability testing, preferably within about 48 hours.

The reference portions may in some embodiments comprise whole plants, but typically the reference portions comprise one or more segments of a root or rhizome system of the same species as the plant or plants to be treated; for example Japanese Knotweed.

The reference portions may suitably be contained in moist container media (e.g. perlite or peat). The container medium should preferably be of relatively low conductivity. The reference portions and container media may suitably be contained in a retrieval bag such for example as a breathable polythene bag. Typically, a retrieval bag may contain 1-5 reference portions of the plant to be treated, e.g. 2 or 3 portions.

Where lethal treatment is carried out *ex situ*, e.g. by excavating contaminated soil from a site and transporting it to a different location for treatment, one or more bagged reference portions may be added to the soil prior to and optionally during application of the lethal treatment to the soil. One retrieval bag may be used per 50-150 m$^3$ soil, e.g. per 100 m$^3$. The viability of the reference portions may be checked at the end of treatment and, optionally, one or more times periodically during treatment.

Where the lethal treatment is carried out *in situ*. i.e. at the site where the unwanted plant or plants are growing, one or more retrieval bags can be included at spaced locations and at different levels to investigate treatment differences over depth to provide a 3-D interpretation. Suitably, at each location, a well casing, for example a metal well casing, may be driven into the subsurface, with one or more bagged reference portions placed within the casing at different depths; e.g. one bag per 0.1-1.0 m. A plurality of retrieval bags may be buried at intervals of 0.5 m depth, for example. Again, the viability of the reference portions may be checked at the end of treatment and, optionally, one or more times periodically during treatment

Suitably, one or more probes may be buried in or adjacent the retrieval bags. For example, non-wired probes or loggers may be included within the bag or bags. For *in situ* treatment, one or more probes may be included inside the well casing, again optionally at different depths.

The probe or each probe may comprise a temperature probe. Advantageously, emplacement of probes (e.g., temperature probes) with the reference portions being buried allows the exposure of the treatment effect to the buried reference portions to be directly correlated. Parallel monitoring of lethal effect (e.g., temperature) is not only a synergy, but also provides direct linkage of treatment effect and plant outcome.

Monitoring the status of the buried reference portions in the retrieval bags allows the effectiveness of an ongoing *in situ* or *ex situ* lethal treatment to be examined. As a control, one or more reference samples in retrieval bags may be placed outside a treatment area.

Thus, determining the effectiveness of the lethal treatment in accordance with a method of the invention, may comprise assessing the status of the one or more reference samples after application of the lethal treatment to the plants or groups of plants to be treated. The reference samples are subjected to the same treatment as the plants or groups of plants to be treated, and thus provide a good indicator of the effectiveness of the treatment. Of course, the viability of one or more plants or groups of plants which are native to the site to be treated where they are unwanted may also be checked directly using methods in accordance with the present invention.

According to a third aspect of the invention there is provided a method of validating a treatment for killing a plant having a root or rhizome system, the method comprising;

i) determining the $CEL_{sample}$ of at least one first sample of the root or rhizome system of a specimen of the plant by:

a) measuring the electrolyte leakage of the first sample ($EL_{sample}$);
b) exposing the first sample to cell barrier disrupting conditions;
c) measuring the electrolyte leakage of the first sample ($EL_{total}$) after the sample has been exposed to the cell barrier disrupting conditions; and
d) calculating the comparative electrolyte leakage of the first sample ($CEL_{sample}$):

$$CEL_{sample} = 100 \times \left( \frac{EL_{sample}}{EL_{total}} \right)$$

ii) applying a treatment to the specimen;
iii) determining the $CEL_{sample}$ of at least one second sample of the root or rhizome system of the specimen after treatment; and
iv) comparing the $CEL_{sample}$ of the first and second samples before and after the treatment to determine the effectiveness of the treatment applied.

In some embodiments, the first and second samples may be taken from the same or different portions of the root or rhizome system of the specimen of the plant.

In some embodiments, the treatment may be applied to a portion of the specimen of the plant, such for example as a rhizome segment, which has been separated from the rest of the specimen. In such case, the at least one second sample should be taken from the treated portion of the specimen.

Such a method may be particularly advantageous for determining the effectiveness of a treatment in control plots or in laboratory scale experiments, to determine the treatment's suitability for killing plants, especially when the treatment is applied to root or rhizome systems of such plants. Optionally, roots or rhizomes, or portions of roots or rhizomes, may be isolated in a sealed container before a treatment is applied. A treatment may then be applied to the container, for example a heat treatment, so that all roots or rhizomes, or portions of roots or rhizomes, are subjected to the same treatment. After treatment, samples of the roots or rhizomes are obtained and the $CEL_{sample}$ of the sample(s) determined to determine the effectiveness of the treatment. Optionally, the sealed container may be buried before the treatment stage, for example in soil. Optionally, the sealed container may be buried together with probes to monitor the treatment, for example one or more temperature probes. The probes may be buried within the sealed container or outside of the sealed container but in close proximity to the sealed container; for example within about 5 cm of the sealed container.

According to a fourth aspect of the invention there is provided a method for optimising a heat treatment for killing a plant, the method comprising;

i) determining if a portion of the root or rhizome system of a specimen of the plant is viable by:

a) measuring the electrolyte leakage of at least one sample of the portion of the root or rhizome system ($EL_{sample}$);
b) exposing the sample to cell barrier disrupting conditions; and,
c) measuring the electrolyte leakage of the sample ($EL_{total}$) after the sample has been exposed to the cell barrier disrupting conditions; and
d) calculating the comparative electrolyte leakage of the sample ($CEL_{sample}$):

$$CEL_{sample} = 100 \times \left( \frac{EL_{sample}}{EL_{total}} \right)$$

ii) applying heat treatment to the same or a different portion of the root or rhizome system of the specimen plant for a defined period of time and at a defined temperature;
iii) determining if the heat-treated portion has been killed by the heat treatment by:

a) measuring the electrolyte leakage of at least one sample of the heat-treated portion ($EL_{sample}$);
b) exposing the sample to cell barrier disrupting conditions; and
c) measuring the electrolyte leakage of the sample ($EL_{total}$) after the sample has been exposed to the cell barrier disrupting conditions; and,
d) calculating the comparative electrolyte leakage of the sample ($CEL_{sample}$):

$$CEL_{sample} = 100 \times \left( \frac{EL_{sample}}{EL_{total}} \right)$$

iv) repeating steps ii) and iii) on one or more further portions of the root or rhizome system of the specimen plant, wherein each time step ii) is repeated a different period of time and/or temperature is used, and
v) determining the optimum heat treatment by comparing the $CEL_{sample}$ values obtained for the different portions of the root or rhizome system.

Optionally, roots or rhizomes, or portions of roots or rhizomes, may be isolated in a sealed container before a heat treatment is applied. A heat treatment may then be applied to the container so that all roots or rhizomes, or portions of roots or rhizomes, are subjected to the same treatment. After heat treatment, samples of the roots or rhizomes may be obtained and the $CEL_{sample}$ of the sample(s) determined to determine the effectiveness of the treatment. Optionally, the sealed container may be buried before the heat treatment stage, for example in soil. Optionally, the sealed container may be buried together with probes to monitor the heat treatment, for example one or more temperature probes. The probes may be buried within the sealed container or outside of the sealed container but in close proximity to the sealed container, for example within about 5 cm of the sealed container.

Step v) may be performed in accordance with the first aspect of the invention. Additionally, $CEL_{sample}$ of the samples may be compared against C1 and/or C2 according to the first aspect of the invention. It will be understood that in some embodiment, a mean $CEL_{sample}$ value of a plurality of replicate samples may be measured at each stage, and the optimum heat treatment assessed by comparing the mean $CEL_{sample}$ values for the samples with C1 and/or C2.

Such a method may be especially advantageous in determining the effectiveness of a heat treatment in control plots or in laboratory scale experiments, to determine such heat treatment's suitability for killing plants, especially when the treatment is applied to root or rhizome systems of such plants. According to this method, the $CEL_{sample}$ or mean $CEL_{sample}$ values obtained for samples treated at different temperatures and/or for different periods of time may be compared, so that optimum conditions can be determined. Such optimum conditions may be those which require a minimum temperature applied for a minimum amount of time to kill the samples effectively. In terms of time and cost efficiencies, it may be advantageous to obtain the minimum temperature and/or time thresholds which are needed to treat or kill a plant reliably.

It will be understood by the those skilled in the art that analysis of measured $CEL_{sample}$ or mean $CEL_{sample}$ values obtained for a plurality of samples in accordance with any of the foregoing aspects of the invention may conveniently be performed remotely from a site where the $CEL_{sample}$ values are measured. The present invention thus comprehends transmitting measured $CEL_{sample}$ or mean $CEL_{sample}$ values in electronic form (i.e. as data) to a remote location for analysis. Measured $CEL_{sample}$ or mean $CEL_{sample}$ values may for example be uploaded to a remote server for processing via the Internet or another suitable digital communications system. Similarly, the results of analysis, i.e. an assessment of the status of a plant or group of plants, the effectiveness of a certain treatment for killing a plant or data representing optimised parameters for killing a plant may be transmitted back in electronic form to the site where the $CEL_{sample}$ or mean $CEL_{sample}$ values were measured, or to a different location. Carrying out analysis at a central location for data received from multiple different sites may allow a data-set to be collected of $CEL_{sample}$ values for a plurality of different plants, plants grown under different conditions or the like, to permit further improvements in the methods of the invention.

According to a fifth aspect of the invention therefore, there is provided a computer-implemented method of obtaining a determination of the effectiveness of a lethal treatment which has been applied to a plant or a group of two or more plants of the same species or variety in accordance with a method according to the first aspect of the present invention; the method comprising the steps of;

> i) at a local computer, transmitting an electronic signal encoding input data representing one more individual $CEL_{sample}$ values or a mean $CEL_{sample}$ value of one or more root or rhizome samples taken from the treated plant or plants to a remote computer via a data communications network for processing; and
>
> ii) at the same or a different local computer, receiving from the remote computer an electronic signal encoding data output representing an assessment of whether the treatment was successful in killing the plant or plants; wherein the assessment is made at the remote computer on the basis of the $CEL_{sample}$ values or mean $CEL_{sample}$ value.

In this way, the $CEL_{sample}$ or mean $CEL_{sample}$ determined from a plant, or one or more plants, which has been treated can be transmitted to a remote computer, and an assessment received via the remote computer on the basis of $CEL_{sample}$ or mean $CEL_{sample}$.

Preferably, according to the computer-implemented methods of the invention, the output data may be transmitted to a different computer as an electronic signal encoding the output data, or displaying a readable representation of the output data on a display. Thus, a third party may obtain the assessment without having to process the $CEL_{sample}$ or mean $CEL_{sample}$ to obtain such an assessment.

According to a sixth aspect of the invention is provided a computer-implemented method of making an assessment of the effectiveness of a lethal treatment applied to one or a group of two or more plants in accordance with a method according to the first aspect of the invention; the method comprising the steps of;

> i) at a computer, receiving an electronic signal encoding input data that represents one more individual $CEL_{sample}$ values or a mean $CEL_{sample}$ value of one or more root or rhizome samples obtained from the treated plant or plants; and
>
> ii) at the computer, processing the input data to assess whether the treatment has been successful on the basis of the $CEL_{sample}$ values or mean $CEL_{sample}$ value and encoding the result of the assessment as output data.

Preferably, the $CEL_{sample}$ or mean $CEL_{sample}$ value may be compared to a database of $CEL_{sample}$ or mean $CEL_{sample}$ values to determine whether the plant is dead and the treatment is successful, or if the plant is not dead, or the plant's status is indeterminate, and the treatment has not been successful.

According to a seventh aspect of the invention is provided a computer-implemented method of obtaining an assessment of the effectiveness of a lethal treatment applied to one or a group of two or more plants in accordance with a method according to the first aspect of the invention; the method comprising the steps of;

> i) at a first computer, transmitting an electronic signal encoding input data representing a $CEL_{sample}$ value or mean $CEL_{sample}$ value obtained from the treated plant or plants to a remote second computer via a data communications network for processing; and
>
> ii) at the second computer, receiving the electronic signal encoding input data and processing the input data to assess on the basis of the $CEL_{sample}$ value or mean $CEL_{sample}$ value whether the treatment has been successful and encoding the result of the assessment as output data.

Optionally, the computer-implemented method further may comprise at the first computer or a third computer, receiving from the second computer the electronic signal encoding data output representing an assessment of whether the treatment was successful in killing the plant or plants.

Optionally, in accordance with the computer-implemented methods of the invention, the system may trigger human intervention if the status of a sample cannot be automatically determined by processing of the input data on a computer, for example if the CEL$_{sample}$ or mean CEL$_{sample}$ value is borderline between that of a live or dead sample. A human may then input their assessment manually into the computer.

The CEL$_{sample}$ or mean CEL$_{sample}$ values for use in the computer-implemented methods of the fifth, sixth and seventh aspects of the invention may suitably be measured as described elsewhere herein, but for the sake of clarity, it will be understood that in general a CEL$_{sample}$ value may be obtained for a plant or group of plants which have been treated by:

a) measuring the electrolyte leakage of at least one root or rhizome sample (EL$_{sample}$) taken from the one or more plants after the one or more plants have been treated;
b) exposing the sample to cell barrier disrupting conditions;
c) remeasuring the electrolyte leakage of the sample (EL$_{total}$), after the sample has been exposed to the cell barrier disrupting conditions; and
d) calculating the comparative electrolyte leakage of the sample (CEL$_{sample}$):

$$CEL_{sample} = 100 \times \left(\frac{EL_{sample}}{EL_{total}}\right).$$

In general, it will be appreciated unless stated or implied otherwise that features described in relation to one aspect of the present invention may also be incorporated in other aspects of the present invention. For example, the methods of determining the effectiveness of a lethal treatment of the invention may incorporate any of the features described with reference to the methods of treatment of an invasive plant species and *vice versa.*

Description of the Drawings

Embodiments of the present invention are described below by way of example only with reference to the accompanying schematic drawings in which:

Figure 1a is a photograph showing a lignified rhizome of Japanese knotweed of a suitable size and shape for obtaining samples for ELT testing.
Figure 1b is a photograph showing cutting off slices of rhizome suitable for testing by the ELT method
Figure 1c is a photograph showing originally live ("fresh") and dead (as created in the laboratory) lignified rhizome of Japanese knotweed in Universal vials during CEL measurement, after a 16 hour incubation period (left image) and after subsequent heating at 90 °C for 20 minutes (right image);
Figure 2 is a histogram of all CEL$_{sample}$ values (%), with live samples to the left (identified with upward diagonal hatch) of the graph and dead samples to the right (identified with downward diagonal hatch), showing fitted normal distributions (solid and dashed lines). A clear separation is shown between the mean CEL$_{sample}$ values of live and dead samples. Samples falling within the intermediate region may be reanalysed in order to try and reach a definitive conclusion on the status of the sample.
Figure 3 shows site mean CEL$_{sample}$ values for live and dead treated Japanese knotweed rhizomes taken from ten different sites, with error bars showing the 95% confidence interval range.
Figure 4a is a histogram and summary statistics for CEL$_{sample}$ values for live Japanese knotweed rhizome samples from site 6 of Figure 3.
Figure 4b is a histogram and summary statistics for CEL$_{sample}$ values for dead treated Japanese knotweed rhizome samples from site 6 of Figure 3.
Figure 5a is a photograph of a piece of live lignified rhizome of Japanese knotweed which has been incubated on agar get for six days, on which fresh roots have started to grow.
Figure 5b is a photograph of a piece of dead lignified rhizome of Japanese knotweed on which decay has started, as indicated by fungal growth.
Figure 6 is a bar chart of CEL$_{sample}$ value of samples of lignified rhizome from Japanese knotweed determined after the samples of lignified rhizome were heated at a range of different respective temperatures from 40 °C to 60 °C for between 1 and 4 hours. Each individual experiment was replicated at least once. Heating the sample at 2 hours at 50 °C was found to be sufficient to kill a sample and was sufficient to create a significant difference (>2 SD) between the CEL$_{sample}$ values of this treatment and less vigorous treatments.
Figure 7 is a bar chart of CEL$_{sample}$ value of samples of lignified rhizome from Japanese knotweed determined after the samples of lignified rhizome were heated at a range of different respective temperatures from 40 °C to 50 °C for between 1 and 4 hours. Heating the samples at 50 °C for 2 hours was sufficient to kill the samples when assessed by ELT.

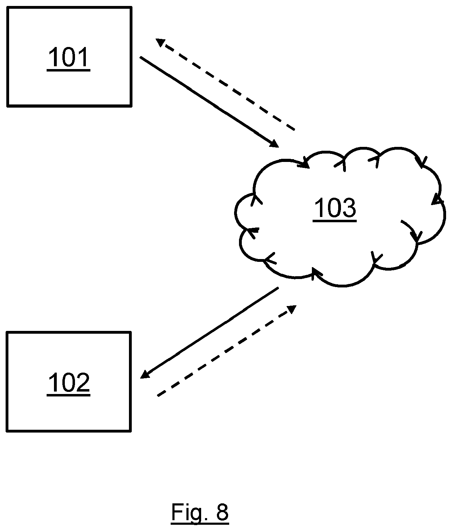

Figure 8 shows a computer-implemented method of the invention. A first computer 101 transmits a signal encoding input data representing a $CEL_{sample}$ value obtained from a plant that has been treated to a remote computer via a data communications network 103 for processing. A second computer 102 receives the signal encoding input data and processes the input data to make an assessment on the basis of the $CEL_{sample}$ of whether the treatment has been successful. The second computer 102 encodes the result of the assessment as output data. The output data is then transmitted to the first computer 101 and is displayed on the screen of the first computer 101 as a readable representation of the output data on a display.

Figures 9a and 9b are fluorescent images of Japanese knotweed lignified rhizome stained with fluorescein acetate. Both fresh and dead (heat treated) samples were analysed. The bright regions in the images show areas of fluorescence. No consistent pattern of fluorescence was observed between live and dead samples.

Figure 10 shows absorbance values obtained by colorimetry for multiple samples of dead and live Japanese knotweed rhizome stained with 2,3,5-triphenyltetrazolium chloride (TTC). Samples were stained with TTC and their absorption measured, which shows the number of samples falsely identified as live or dead was unacceptably high for use as a practical validation method of the effectiveness of a treatment.

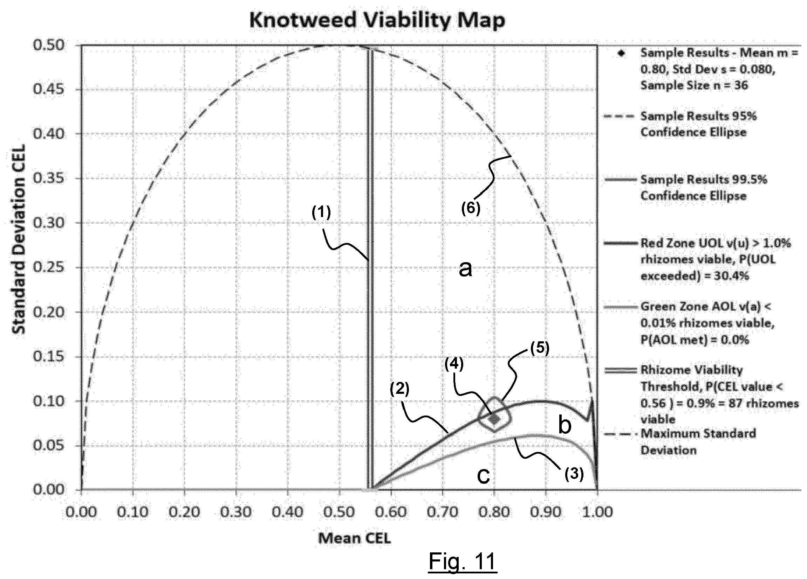

Figure 11 shows a viability map which allows convenient interpretation of a mean $CEL_{sample}$ value determined from a sample based analysis. Mean $CEL_{sample}$ value was determined from a proportion of the total rhizomes available from a site. The viability map is a plot of mean $CEL_{sample}$ value against standard deviation of $CEL_{sample}$ value. C1 is indicated by vertical line (1). Mean $CEL_{sample}$ values lying to the left of C1 (i.e mean $CEL_{sample}$ value <0.56) indicate that there are viable (alive) rhizome samples within the sampled population. Mean $CEL_{sample}$ values lying to the right of C1 (i.e mean $CEL_{sample}$ value >0.56) indicate that there are non-viable (dead) rhizome samples within the sampled population. Line (2) indicates an Unacceptable Outcome Level (UOL). A mean $CEL_{sample}$ value lying on or above the UOL line (2) indicates that an unacceptable number of rhizomes of the plant or group of plants to which a lethal treatment has been applied are viable (i.e. >1.0% of rhizomes are viable). Line (3) indicates an Acceptable Outcome Level (AOL). A $CEL_{sample}$ values lying on or below the AOL line (3) indicates that an acceptable number of rhizomes of the plant or group of plants to which a lethal treatment has been applied are viable (i.e. <0.01% of rhizomes are viable).

Detailed Description

In some implementations of the present invention, one or more plants may be treated to kill the one or more plants and then one or more samples of the treated one or more plants are analysed in order to verify that the lethal treatment has been successful (the plant is dead) or if the plant requires further treatment (the plant is still alive). Such methods may be generally categorised as chemical, physical, biological or mechanical. Methods of lethal treatment which may be used in accordance with the present invention include pesticides, herbicides, chemical agents, composting, stem injection of a herbicide and thermal treatments such as steam or hot air treatment, electrical treatment and pH treatment. These methods are further described below.

In some embodiments, the invention further comprehends testing the viability of a plant before a lethal treatment is applied. This may be used *inter alia* to determine if a lethal treatment is in fact necessary. For example, it can be difficult to determine whether a plant which has lain dormant over winter is dead or alive. Electrolyte leakage testing (ELT) methods (as described herein) may be applied to the plant before lethal treatment in order to determine whether the plant is alive or dead, and therefore whether treatment is required.

Stem injection of herbicide

Stem injection treatment methods are considered to be an effective and environmentally-friendly treatment method for killing invasive plant species, such as Japanese knotweed. Such methods are disclosed by WO 2005/004583 A1 and GB 2,384,158 A, the contents of which are fully incorporated herein by reference. A small dose of herbicide, typically a glyphosate herbicide, is injected into the stem of a plant. Injection of the herbicide into the stem results in a faster rate of absorption than would result from foliar spraying, reduces the risk of contaminating surrounding vegetation and is safe to use near water. Stem injection is not impacted by wind, rain or convection and can be used at any time during a growing season.

Heat treatment

Heat or thermal treatments may be used to kill invasive plant species such as Japanese knotweed.

Thermal treatments may be used to increase the temperature of the subsurface of soil. Such treatments may also be applied to soil excavated from a site. However, a benefit of *in situ* thermal treatment is that it avoids excavation of the soil. In some embodiments, soil may be treated by one or more of the following methods: surface heating via heating blankets; steam/hot water injection; hot air treatment; electrical conductive heating; thermal conductive heating, typically using one or more electrically heated elements or heating wells heated by combustion gases; *in situ* flushing with hot gases, water or steam; venting with steam or heated air; irradiation by radio-frequency, infra-red or microwave electromagnetic radiation; mobile systems in which steam and hot air are injected through blades attached to a rotating shaft. In some embodiments, soil heat treatment may advantageously be conducted beneath existing buildings or other constructions. A further approach is to use smouldering, in which a subsurface combustible organic substance is ignited (typically hydrocarbons or polyaromatic hydrocarbons) with a controlled injection of air into the subsurface. This approach relies on a self-sustaining exothermic process of gradual combustion of the subsurface organic substance.

One such treatment which may be used to kill invasive plant species, such as Japanese knotweed, is soil steam sterilisation, also referred to as soil steaming: Soil is treated with steam or superheated steam (at about 180 - 200 °C) which kills/denatures bacteria, viruses, fungi, nematodes and other pests and also weeds, weed seeds and weed rhizomes and root systems. Steam treatment may be applied at different soil depths; for example surface steam treatment or deep soil treatment. Suitably, and in particular when the invasive plant species being treated is Japanese knotweed, the soil may be treated to a depth of at least about 0.25 m, or at least about 0.5 m, or at least about 1 m, or at least about 2 m. Deep treatment of the soil advantageously reduces the risk of new seeds, roots or rhizomes, being brought to the surface; for example through digging or ploughing.

Alternatively or additionally, in some embodiments, targeted steam treatment, may be used. This allows focused steam treatment for example of root or rhizome systems of plants.

Generally, steam treatment may be performed using methods known to those skilled in the art; for example, sheet steaming, depth steaming with vacuum or negative pressure, steaming with hoods, combined surface and depth injection of steam (sandwich steaming), or container or stack steaming.

If a site is not suitable for *in situ* soil treatment, then the contaminated ground may be excavated, for example to a depth of 2m, and the soil heated *ex situ* in piles or bunds to kill invasive plant species. Alternatively, or additionally, the soil may be placed in insulated containers which are adapted to apply a heat treatment to the soil. For example, the container may contain a means for generating heat. The treated soil may then be placed back into the excavation site or otherwise disposed of.

Suitably, hot air treatments may be used where hot air is passed through the soil. Hot air treatments used in the methods of the present invention may involve heat treating the soil at a temperature of at least about 50 °C, at least about 60 °C, at least about 70 °C, at least about 80 °C or at least about 90 °C. Suitably, the soil may be treated for at least about 30 minutes, at least about 60 minutes, at least about 90 minutes or at least about 120 minutes at one of those temperatures, or a combination of temperatures, for example a lower temperature followed by a higher temperature.

Additionally or alternatively, steam treatments used in the methods of the present invention may involve heat treating the soil. The soil may be heated at a temperature of at least about 50 °C, at least about 60 °C, at least about 70 °C, at least about 80 °C or at least about 90 °C. Suitably, the soil may be treated for at least about 30 minutes, at least about 60 minutes, at least about 90 minutes or at least about 120 minutes at one of those temperatures, or a sequence of different temperatures; for example a lower temperature followed by a higher temperature. Steam treatments may be preferred over hot air treatments because steam has a higher heat capacity of steam compared to air means that lower volumes of steam are required to heat the soil to a given temperature compared to the volume of air needed. However, use of steam may cause leaching and thus add leachate management costs to the treatment. The most suitable type of treatment will depend on the site to be treated and thus is assessed on a case by case basis.

Thermal insulation of the soil to be treated may be required to prevent significant heat losses from the surface. A thermal barrier may be provided by a geo-membrane placed over the surface of the soil being treated.

If necessary, biological activity may be re-introduced to the soil following heat treatment. This may be particularly beneficial where soils are to be re-used.

Optionally, where an *in situ* heat treatment is used as a treatment, a temperature probe may also be buried in the soil adjacent to the plant being treated, for example within 50 cm of the plant being treated. Thus, the temperature of the soil surrounding the plant can be measured. It is then possible to determine that the plant has received a heat shock of sufficient temperature and for a sufficient length of time to kill the plant.

Electrolyte leakage testing (ELT)

The methods of the present invention utilise electrolyte leakage (EL), also referred to as comparative electrolyte leakage (CEL), for assessing the status of roots or rhizomes, such as those of Japanese knotweed, after one or more plants has been treated according to the methods described herein.

The ELT method may be simple and easy to perform and requires a sample of plant root or rhizome to be submerged in de-ionised water and the conductivity of the water measured. In live samples, the electrolyte (solutes) of live cells is contained by intact cell barriers and therefore does not escape, and a low conductivity of the water surrounding the sample will be measured. However, heating the sample, or otherwise subjecting the cell to harsh conditions, disrupts or damages the cell barriers , increasing the permeability of the cells and thus leaking electrolyte into the surrounding solution. Electrolyte leakage is therefore a function of membrane/cell wall permeability, and is sometimes termed conductivity, as it is the measure of the conductivity of a solution in which plant material has been placed.

In accordance with the present invention, a plant from which a sample was obtained can be determined as alive or dead, and thus the effectiveness of a lethal treatment applied to said plant may be determined using the ELT method. If the plant has been killed by the treatment, then the sample obtained will be dead and thus the cell barriers will already have lost their ability to effectively hold electrolytes within the cell. Submerging the sample in de-ionised water would therefore give a relatively high conductivity reading. Heating the sample and then re-measuring the conductivity of the solution will result in relatively little change in EL value since the cell barriers were already disrupted before heating the sample.

However, in the case of a live plant, the initial conductivity measured after the sample is submerged in water at room temperature will typically be low. Heating said sample will result in significant cell barrier disruption and thus a significant increase in EL compared to before the sample was heated. It is this change in electrolyte leakage which occurs after heating the sample which can be used to determine the status of the parent plant.

Root electrolyte leakage (REL) methods have been used previously to determine the viability of roots of tree species grown in nurseries. However, REL has not hitherto been used for determining the effectiveness of a lethal treatment applied to weeds and invasive plant species, in particular Japanese knotweed. (McKay H.M. 1998. Root electrolyte leakage and root growth potential as indicators of spruce and larch establishment, Silva Fennica 32(2), 241-252).

It is known in the art to use REL for testing "fine" roots (typically specified as < 2 mm diameter) of horticultural plant species. However, such test methods have only been applied to soft roots and rhizomes; not to lignified rhizomes. Further, such previous REL testing methods have only been used for determining the viability of horticultural plants, typically trees and grass rhizomes, to determine the viability of the plants after storage for a period of time, or in recovery from damage. Typically, samples are taken from nurseries under managed conditions and sampled plants are young or at seedling stage, and no more than 4 years old.

Previous testing methods for determining REL measurements are not applicable to lignified rhizomes, such as those obtained from Japanese knotweed. Japanese knotweed rhizomes are not suitable, for example because they are typically much larger than 2 mm in diameter and need to be cut to size to be dealt with practically, but care also needs to be taken to have a representative sample as explained below.

The methods of the present invention allow samples of roots or rhizomes to be tested as to whether they are alive or dead with over about 95% reliability. Advantageously, the testing methods of the invention may be combined with a lethal treatment of the plant, such for example as a lethal treatment which is designed to kill the plant, to determine the effectiveness of the lethal treatment. The present methods are applicable to samples taken from field sites, as well as managed systems. The methods of the present invention may also be applied to other invasive plant species or weeds.

The methods of the present invention may be used for determining the effectiveness of a lethal treatment applied the roots or rhizomes of a range of plant species and varieties, such as Japanese knotweed. A sample of root or rhizome is obtained from a plant which has already been treated. Suitably, one or more samples may be obtained per plant.

For plant species having rhizome networks, advantageously only thin slices of relatively thick rhizome are required, rather than long segments of fine root, to provide enough sample for analysis. The sample(s) obtained are suitably cut from the rhizome of the plant and may have a thickness of between about 1 mm and about 3 mm; preferably the thickness is about 2 mm. The samples may be obtained from a rhizome having a diameter of between about 5 mm and 50 mm to allow for easy handling. The rhizome selected should be free from obvious signs of damage or disease.

Once obtained, the sample is then submerged in deionised water in a suitable receptacle, and the sample is stored for a prolonged period of several hours, for example overnight, to allow the conductivity of the solution to equilibrate. Preferably, the sample should be fully submerged in the deionised water. Where multiple samples are tested, they should each be placed in deionised water in individual receptacles and tested separately under similar conditions.

The conditions should be chosen so as not cause any significant damage to the sample. For example, the sample may be stored at room temperature (approximately 15 °C to 25 °C). The sample may be stored in the dark, with the exclusion of light. The sample may be stored for several hours; typically at least 5 hours or at least 10 hours. Preferably, the sample may be stored for about 16 hours or overnight. In some embodiments, the sample may be stored for up to about 24 hours.

The conductivity of the solution is then measured using methods which are well known to those in the art; for example using a conductivity probe. The higher the conductivity, the more electrolyte has diffused through the cell membrane into the solution surrounding the sample. Therefore, the greater the absolute conductivity of the solution, the greater the damage to the cell barriers of the sample. The electrolyte leakage of the sample is called the $EL_{sample}$.

The sample is then exposed to elevated temperatures. For example, the sample may be heated at about 50 °C, about 70 °C, about 90 °C, about 100 °C or any intermediate temperature, for a period of at least 10 minutes, at least 20 minutes or at least 30 minutes. It will be appreciated that any lethal treatment that causes the disruption of the cell barriers is suitable for use in addition to or as an alternative to heat treatment. Such conditions are herein referred to as cell barrier disrupting conditions. For example, additional or alternative conditions may include heat treatment and/or exposure to at least one of sonication, ionising (e.g. gamma) radiation and microwave radiation.

The electrolyte leakage of the sample is then measured after the sample has been exposed to such cell barrier disrupting conditions. The measured electrolyte leakage of the sample after being exposed to cell barrier disrupting conditions is called $EL_{total}$. If the sample was previously alive before exposure to the cell barrier disrupting conditions, then the electrolyte leakage of the sample will have increased significantly as compared to the electrolyte leakage of the sample tested before exposure to cell barrier disrupting conditions, as the cell barrier disrupting conditions will have caused damage to the cell membrane and/or wall and thus increased the leakage of electrolyte out of the cell. If the sample was already dead before exposure to the cell barrier disrupting conditions, then only a small increase in electrolyte leakage of the sample will occur after exposing the sample to cell barrier disrupting conditions as the cell membranes and walls will already have been damaged before the sample was exposed to cell barrier disrupting conditions. The change in electrolyte leakage following exposure to cell barrier disrupting conditions can therefore be used to determine whether the original sample was alive or dead. This in turn provides information on the status of the parent plant and consequently informs whether a lethal treatment applied to that plant has been successful or not.

The comparative electrolyte leakage of an individual sample, $CEL_{sample}$, is used to determine the change in electrolyte leakage of a sample when exposed to conditions that disrupt the cell barrier, i.e. the cell barrier disrupting conditions. The comparative electrolyte leakage of a sample is calculated according to the following equation:

$$CEL_{sample} = 100 \times \left( \frac{EL_{sample}}{EL_{total}} \right)$$

The larger the $CEL_{sample}$, the more likely a sample is to be dead, and thus the more likely a lethal treatment applied to the plant has been successful. As it is important to be able to determine accurately and reliably whether or not a treated plant, such as Japanese knotweed, is dead, the $CEL_{sample}$ value may be evaluated to determine with a high level of confidence the status of one or more plants that have been treated. For example, $CEL_{sample}$ may be compared with one more known reference $CEL_{sample}$ values. In some embodiments, $CEL_{sample}$ may be compared with one more critical values. In some embodiments, the one more critical values may be predetermined values, such as thresholds.

In some embodiments, if the $CEL_{sample}$ is above a predetermined critical value (C1) then the sample is assessed to be dead and the lethal treatment is deemed to have been effective. Conversely, if the $CEL_{sample}$ is less than the critical value (C1) then the sample is assessed to be not dead and the lethal treatment is deemed to have been ineffective.

A suitable method for calculating the critical value (C1) comprehends measuring $CEL_{sample}$ of one or more known-to-be dead and live reference samples taken from rhizomes or roots of the plant species to be treated. Preferably the $CEL_{sample}$ values of a plurality of known-to-be dead and live reference samples are measured. The critical value (C1) may be selected as a $CEL_{sample}$ value that discriminates between the live and dead reference samples with ≥95% accuracy.

In some embodiments, a database of known $CEL_{sample}$ values of a plurality of known-to-be dead and live reference samples may be created. The critical value (C1) may be calculated as the average of the $CEL_{sample}$ values of the plurality of live and dead reference samples in the database.

The dead and live reference samples may be obtained from the same plant. For example, two samples may be obtained from a plant which is known to be alive. One sample is referred to as the live reference sample. The second sample is killed, for example by exposing it to a heat treatment such as heating at about 50 °C for about 2 hours, and is referred to as the dead reference sample. The $CEL_{sample}$ values of both the dead and live reference samples are then determined.

That is, the electrolyte leakage of each of the live and dead reference samples may be measured ($EL_{sample}$) by submerging the samples individually in deionised water and measuring the conductivity of the solutions, as described above. Each of the live and dead reference samples is then exposed to cell barrier disrupting conditions. These conditions may include heat treatment and/or exposure to one or more of sonication, microwave radiation and ionising (e.g. gamma) radiation. Various conditions may be used to treat the samples. For example, the samples may be heated at about 50 °C, about 60 °C, about 70 °C, about 90 °C, or about 100 °C for a period of at least 10 minutes, at least 20 minutes or at least 30 minutes. The live and dead samples are treated in the same way.

Following exposure to cell barrier disrupting conditions, the electrolyte leakage of each of the dead and live reference samples ($EL_{total}$) is determined after the reference samples have been exposed to cell barrier disrupting conditions. Again, this is done by submerging the samples individually in deionised water and measuring the conductivity of the solutions. The $CEL_{sample}$ of the each of the dead and live reference samples is then determined according to the following equation:

$$CEL_{sample} = 100 \times \left(\frac{EL_{sample}}{EL_{total}}\right)$$

Advantageously, the $CEL_{sample}$ of a plurality of live and dead reference samples may be determined. For example, at least 10 live and 10 dead reference samples may be analysed; or at least 50 dead and 50 live reference samples or even as many as 100 or more dead and 100 or more live reference samples may be analysed. Suitably, the live and dead reference samples may be obtained in pairs so that each one of the one live reference samples comes from the same plant as a corresponding one of the dead reference samples. Herein, the term "reference sample" refers to a sample taken from a plant which is known to be alive or dead and for which the $CEL_{sample}$ is calculated in order to determine a reference value (the critical value, C1) to which other samples of unknown status can be compared. For the avoidance of doubt, the reference sample is not the same as the sample obtained from a plant that has been treated.

These conditions ensure that the $CEL_{sample}$ values of live and dead reference samples of plants, which have grown under different conditions and/or in different environments, are measured.

The reference samples are obtained from the same plant species or variety which is the subject of the lethal treatment. Thus, when the methods of the invention are used for determining the effectiveness of the lethal treatment of Japanese knotweed, the one or more reference samples should also be obtained from Japanese knotweed.

Once a plurality of $CEL_{sample}$ values have been determined for the dead and live reference samples, the critical value (C1) may be selected as a $CEL_{sample}$ value that discriminates between the plurality of live and dead reference samples with ≥95% accuracy. The $CEL_{sample}$ of the live and dead reference samples may be stored in a database to which a user may refer to determine the most suitable critical value (C1) for a particular site and/or plant species or variety.

In some embodiments, the critical value (C1) is calculated as the average of the $CEL_{sample}$ +2SD of the live reference samples and $CEL_{sample}$ -2SD of the dead reference samples. This critical value may then be used to determine whether a test sample is alive or dead according to the methods described above. This allows a plant to be determined as dead to at least 95% accuracy.

In some embodiments, particularly in those embodiments where higher accuracy is required, C1 is calculated as the average of the $CEL_{sample}$ -2SD of the dead reference samples only. This allows the plant to be determined to be dead to at least 95% accuracy and preferably greater than 95% accuracy.

The $CEL_{sample}$ of the sample may optionally be compared to a second critical value (C2). If the $CEL_{sample}$ is less than the second critical value (C2), then the sample of rhizome or roots is assessed to be live and treatment to have been ineffective. Suitably, the second critical value (C2) may be equal to the mean $CEL_{sample}$+2SD of a plurality of live reference samples taken from rhizomes or roots of the plant species to be treated. It will be appreciated that the ranges greater than C1 and less than C2 are non-contiguous.

Thus, if the $CEL_{sample}$ of the sample is a value between C1 and C2, then the status of the sample may be assessed to be undetermined and the test therefore inconclusive. In this case, the sample may be retested by the CEL method and/or by a complementary method.

If a number of samples are taken from the one or more plants that have been treated, then it may be advantageous to exclude any outlier $CEL_{sample}$ results and to consider only $CEL_{sample}$ of samples that are representative of the plant, i.e. which will produce a reliable $CEL_{sample}$ value which is consistent with the status (alive or dead) of the plant. For example, a $CEL_{sample}$ result obtained for one sample of a plurality of samples being tested from a plant, that is clearly different from the rest of the $CEL_{sample}$ values obtained from the remaining samples may appropriately be excluded from analysis.

In some embodiments of the invention, a computer-implemented method may be used to transmit a $CEL_{sample}$ value obtained from at least one treated plant to an external network so that an assessment can be made as to the effectiveness of the treatment. An example of such an arrangement is shown in Figure 8. A first computer 101 transmits a signal encoding input data representing a $CEL_{sample}$ value obtained from a plant that has been treated to a remote computer via a data communications network 103 for processing. As an example, the $CEL_{sample}$ value may be uploaded to the computer by a person who has obtained the $CEL_{sample}$ value of a sample from a treatment plant. A second computer 102 receives the signal encoding input data and processes the input data to make an assessment on the basis of $CEL_{sample}$ of whether the treatment has been successful. For example, the second computer 102 may compare the $CEL_{sample}$ value to a database of $CEL_{sample}$ values to make an assessment. The database may contain a plurality of C1 and/or C2 values determined as described herein. The second computer 102 then encodes the result of the assessment as output data. The output data is then transmitted to the first computer 101 and is displayed on the screen of the first computer 101 as a readable representation of the output data on a display. Thus, a person who has uploaded the $CEL_{sample}$ value to the first computer 101 is able to conveniently obtain and view the assessment determined by the second computer 102.

Optionally, the second computer 102 may indicate that human intervention is needed to make an assessment, before encoding the assessment as output data. For example, if the $CEL_{sample}$ value is determined by the second computer 102 to be close to the values C1 and/or C2, such as within 5% of the value of either C1 or C2, then the second computer 102 will indicate to an operator that the assessment needs to be approved by a human operator before the assessment is encoded as output data. In this way, the human operator can override the assessment allocated by the second computer, and the operator can manually input the assessment into the second computer to be encoded as output data.

Complementary methods for determining the effectiveness of a lethal treatment applied to invasive plant species

A complementary method may be employed to determine whether or not a treatment of one or more plants has been effective in case the $CEL_{sample}$ gives an inconclusive result, for example as described above.

One such method comprehends propagation of a sample of rhizome. In accordance with the present invention, this method comprises placing a sample of rhizome obtained from a treated plant on a culture medium (e.g. agar) for an extended period of time of several days, i.e. at least 1 or 2 days; e.g. 5 days, 6 days, 10 days or even 15 or more days. A live sample will sprout root hairs after a period of time. However, if the sample is dead, no growth will be observed and the sample may even exhibit mould growth. The status of a rhizome, as determined by such a propagation method may correlate with the status of the parent plant, and therefore may be used to determine whether or not a treatment has been successful. If this method is chosen, the rhizome sample must contain a node so that growth can occur if the sample is alive.

Such methods may be used in conjunction with the ELT methods of the present invention. It may be particularly advantageous to use such a complementary method for determining the viability of a rhizome in conjunction with the above-described ELT testing methods where the results of the ELT methods are inconclusive.

Methods of validating lethal treatments

The ELT method may be used to validate a method of treatment, for example to refine a lethal treatment or to compare one treatment with another. Such methods may be used on control plots, where samples of live roots or rhizomes are treated on site, or in laboratory experiments, where several samples of root or rhizome, typically from the same plant, may be subjected to different treatments and the $CEL_{sample}$ values of each sample determined and compared to determine the relative effectiveness of the treatments.

The $CEL_{sample}$ of the samples of root or rhizome that have been treated may be determined in the same way as described above; that is, using ELT methods.

The lethal treatments which can be examined may include physical, chemical, mechanical or biological treatments. For example, heat treatments, such as hot air or steam treatments, or herbicide treatments.

Advantageously, these methods may be used to determine the optimal time period, dosage regime or temperature at which a treatment is applied. These methods may also advantageously be used to compare the effect of the same treatment on different plant varieties or species.

Samples of roots or rhizomes may be obtained from roots or rhizomes harvested from a site before and after treatment. The $CEL_{sample}$ value of the root or rhizome samples may be obtained and compared to determine the effectiveness of the treatment applied. This provides an *in situ* method for determining the effectiveness of a treatment. The treatment may be applied by the same carried out by the same, or a different person to that determining the $CEL_{sample}$ value of the sample.

Alternatively, roots or rhizomes may be excavated from a site and a treatment is applied to the root or rhizome *ex situ*. The $CEL_{sample}$ value of the root samples may be obtained and compared before and after treatment to determine the effectiveness of the treatment applied.

$CEL_{sample}$ of samples of root or rhizome used as controls, to which no treatment is applied, may also be determined.

In some embodiments, the treatment may be applied to the plant or roots or rhizome samples obtained from a plant by a third party. Thus, the party determining the $CEL_{sample}$ value is different to the party that applied the treatment to the plant.

Site sampling

Preferably, roots, rhizomes or other plant material or sections thereof are collected from a site by excavation of the soil. Excavation of soil is a particularly effective method of collecting root and/or rhizome material as it allows roots and rhizome systems of plants to be uncovered, even when there is no plant material visible on the surface to indicate the presence of plant material. The collected root and/or rhizome material may then be tested according to the methods presented herein. However, it is often undesirable to sample and test by electrolyte leakage all roots and/or rhizomes that are present at a site. Instead it is typically more time and cost efficient to excavate soil from one or more locations within a site, and analyse only the roots and/or rhizomes uncovered from those locations. Disclosed herein are methods that allow a representative sample of plant material (such as a representative sample of roots and/or rhizomes) to be analysed.

Preferably, only a proportion of plant material available from a plant or group of plants is collected from a site. For example, only a proportion of root and/or rhizome material, or sections thereof are collected from a site. When it is undesirable to sample all root and/or rhizome material present at a site through excavation of the soil, for example if the site has a large volume (e.g. > 200 $m^3$ in volume), the total volume of the site may be divided into smaller volumes (called "excavation units") having a volume less than the total volume of the site. According to this method, only the soil volume represented by the excavation unit(s) is excavated from the site, thus an excavation unit represents the unit volume of soil to be excavated. Multiple excavation units may be distributed across the site. Once selected, an excavation unit is excavated and the soil from the excavation units is processed (for example by sieving the soil through a fine mesh) to uncover the root or rhizome material within the soil. Samples may then be obtained from the collected rhizome and/or root material which may be tested according to electrolyte leakage methods described herein.

The site to be assessed may be defined as having a unit volume of soil. An excavation unit will have a volume less than the unit volume of soil of the site, for example an excavation unit may have less than 1% of the volume of the site, less than 2% of the volume of the site, less than 5% of the volume of the site, or less than 10% of the volume of the site. A convenient way of defining an excavation unit is by the volume of soil that can be excavated by standard soil excavation machinery, such as a digger or excavator. For example, a single excavation unit may have a volume of 1 $m^3$. Typically, between 25 to 30 rhizomes are found per 1 $m^3$ of soil populated with Japanese knotweed.

In order to determine the position or number of excavation units at any given site, it may be convenient to first divide a site (that is a site having a unit volume of soil) into multiple sample units. It will be understood that dividing a site into multiple sample units may be done theoretically, for example by obtaining a map of the site and drawing multiple sample units onto the map of the site. It may be that a computer programme is used to divide the unit volume of soil of a site into multiple sample units, the results of which may be visualised on a map of the site. Each sample unit may have a volume equal to that of an excavation unit. For example, a site having a unit volume of 200 $m^3$ may be divided into 200 sample units having a volume of 1 $m^3$. Only a proportion of the sample units will be excavated. Thus, an excavation unit is equivalent to a sample unit that has actually been, or is intended to be, excavated. For example, only a proportion of the 200 sample units may be excavated from the site and are therefore considered excavation units. Preferably, less than 5, less than 10 or less than 20 sample units may be excavation units and are excavated or intended to be excavated from a site. It may be that less than 50%, less than 40%, less than 30%, less than 20%, or less than 10% of sample units are excavation units. Each sample unit may be allocated a number and a random number generator may be used to select which sample unit(s) is to be allocated as an excavation unit(s).

Although the methods and examples disclosed herein primarily concern collection of root and/or rhizome material through excavation of the soil at a site, it will be understood by the skilled person that such methods could also be applied to collecting plant material found above ground at a site, for example stem and leaf material. Plant material found above the surface of a site may include stem or leaf material, but also includes root or rhizome material that has been brought to the surface (for example through ploughing of the soil). If the plant material on the surface of the soil is to be analysed, the total surface area of the site may be divided into smaller surface areas (called "surface sampling units") having a surface area smaller than the total surface area of the site. According to this method, only the plant material located at the soil surface of the surface sampling unit(s) is collected from the site, thus a soil sampling unit represents a unit area of the site from which plant material will be collected from the surface. Multiple surface sampling units may be distributed across the site. Once selected, the plant material at the surface of the surface sampling unit may be collected and analysed according to the methods described herein.

The site to be assessed may be defined as having a unit surface area. A surface sampling unit will have a surface area less than the surface area of the site, for example an surface sampling unit may have less than 1% of the surface area of the site, less than 2% of the surface area of the site, less than 5% of the surface area of the site, or less than 10% of the surface area of the site. For example, a surface sampling unit may have an area of less than 2 $m^2$, less than 4 $m^2$, less than 6 $m^2$, or less than 10 $m^2$.

In order to determine the position or number of surface sampling units at any given site, it may be convenient to first divide a site (that is a site having a unit surface area) into multiple surface units. It will be understood that dividing a site into multiple surface units may be done theoretically, for example by obtaining a map of the site and drawing multiple surface units onto the map of the site. It may be that a computer programme is used to divide the unit surface area of a site into multiple surface units, the results of which may be visualised on a map of the site. Each surface unit may have an area equal to that of a surface sampling unit. It may be that only the plant material found at the surface of a proportion of the surface units is collected. Thus, a surface sampling unit is equivalent to a surface unit that has actually had the surface plant material collected, or it is intended that the surface plant material will be collected from that unit. Preferably, less than 5, less than 10 or less than 20 surface units may be surface sampling units. It may be that less than 50%, less than 40%, less than 30%, less than 20%, or less than 10% of the surface units are surface sampling units. Each surface unit may be allocated a number and a random number generator used to select which surface unit(s) is to be allocated as a surface sampling unit(s).

Thus, when a plurality of roots and/or rhizomes, other plant material, or sections thereof, are collected from a site, the site may be divided into multiple unit areas (surface unit) or unit volumes (sampling unit), and the plurality of roots and/or rhizomes, other plant material, or sections thereof, are collected from a proportion of the unit areas or unit volumes, as disclosed in the methods herein. Thus, the methods presented herein may comprise first dividing a site into multiple unit areas (surface unit) or unit volumes (sampling unit), selecting a proportion of the unit area and/or unit volumes to be surface sampling units or excavation units wherein root and/or rhizome or other plant material, or sections thereof, are obtained from the surface sampling units or excavation units. Samples may then be taken from the collected plant material which are then analysed according to the methods disclosed herein.

When the plant samples collected from a pre-defined number of excavation units or surface sampling units results in an inconclusive result as to the status (alive or dead) of the plant or plants found at that site following analysis according to the methods disclosed herein, it may be that the site is revisited and plant material is obtained from further excavation units and/or surface sampling units until a conclusive result as to the status of the plant species is reached.

It may be that all plant material, preferably all root or rhizomes material, recovered from the excavation unit(s) and/or surface sampling unit(s) are analysed according to the electrolyte leakage methods presented herein. For example, at least one sample may be taken from each root and/or rhizome collected and analysed by electrolyte leakage testing. Alternatively, a proportion of the plant material collected from the excavation unit(s) and/or surface sampling unit(s) may be analysed according to electrolyte leakage methods presented herein. For example, at least one sample may be taken from a proportion of the total root and/or rhizomes collected and analysed by electrolyte leakage testing. For example, it may be that less than 5%, less than 10%, less than 20% or less than 25% of plant material, such as rhizome or root material, collected from an excavation unit or surface sampling are analysed by electrolyte leakage methods.

Preferably, a trial pit is excavated from a site, more preferably multiple trial pits, such as at least two, more preferably at least three, are excavated from a site. Excavation of multiple trial pits allows identification of any significant difference between the mean $CEL_{sample}$ values determined from each trial pit. Thus, the methods disclosed herein may further comprise calculating a mean $CEL_{sample}$ value on the basis of samples obtained from a first trial pit, calculating a mean $CEL_{sample}$ value on the basis of samples obtained from a second trial pit, and comparing the mean $CEL_{sample}$ values obtained. The method may further comprise identifying whether there is a significant difference in the mean $CEL_{sample}$ values obtained. A trial pit will be understood to be a continuous volume comprising multiple excavation units. For example, a trial pit may comprise 3, 6, 9 or 12 excavation units. The trial pit may be multiple excavation units in length, width and depth, wherein the length and width refer to the surface area of the trial pit and the depth refers to the depth below ground level of the excavation pit. For example, the trial pit may be 2 excavation units in length, 2 excavation units in width, and 3 excavation units in depth.

Preferably, only a proportion of roots and/or rhizomes or other plant material excavated from the trial pit are analysed according to the electrolyte leakage methods disclosed herein. Preferably, samples for electrolyte leakage testing are taken from less than 10, less than 20, less than 30, or less than 40 roots and/or rhizomes obtained per trial pit. It may be that samples for electrolyte leakage testing are taken from between about 10 and about 100, between about 20 and about 60, between about 30 and about 40, or more preferably between about 30 and about 35 of roots and/or rhizomes collected per trial pit. Preferably, it may be that samples for electrolyte leakage testing are taken from less than 10%, less than 20%, less than 30%, less than 40%, or less than 50% of roots and/or rhizomes obtained per trial pit. Preferably, samples for electrolyte leakage testing are taken from at least three roots and/or rhizomes, at least six roots and/or rhizomes or at least ten roots and/or rhizomes randomly selected from each excavation unit of the trial pit. Alternatively, all roots and/or rhizomes excavated from the trial pit are collected and analysed according to the electrolyte leakage methods disclosed herein. A particularly effective way of collecting root and/or rhizome material may be to sieve the soil through a fine mesh. It may be that the root and/or rhizome collected are cut into sections according to the methods disclosed herein and only a proportion of sections are analysed by electrolyte leakage testing.

It may be that the collected rhizomes go through an initial separation process in which rhizomes that are identified as rotting by visual inspection are separated from those rhizomes that are not identified as rotting by visual inspection. This is a particularly quick and easy way of excluding rotten rhizome samples from unnecessary analysis by electrolyte leakage testing. Thus, the rhizomes may be divided into group 1 comprising rhizomes that are identified as rotten by visual inspection and group 2 comprising rhizomes that are not identified as rotten by visual inspection. Preferably, the total number of rhizomes that are identified as rotten by visual inspection (group 1) is determined. Preferably, only the rhizomes that are not identified as rotten by visual inspection (group 2) are analysed by electrolyte leakage testing as disclosed herein. More preferably, only a proportion of roots and/or rhizomes collected that are not identified as rotten by visual inspection (group 2) are analysed by electrolyte leakage testing as disclosed herein. Preferably, it may be that samples for electrolyte leakage testing are taken from less than 10, less than 20, less than 30, or less than 40 roots and/or rhizomes obtained per trial pit. It may be that samples for electrolyte leakage testing are taken from between about 10 and about 100, between about 20 and about 60, between about 30 and about 40, or more preferably between about 30 and about 35 of roots and/or rhizomes collected per trial pit. Preferably, it may be that samples for electrolyte leakage testing are taken from less than 10%, less than 20%, less than 30%, less than 40%, or less than 50% of roots and/or rhizomes obtained per trial pit. Preferably, samples for electrolyte leakage testing are taken from at least three roots and/or rhizomes, at least six roots and/or rhizomes or at least ten roots and/or rhizomes randomly selected from each excavation unit of the trial pit. The rhizomes in group 2 from which at least one sample will be taken to be analysed by electrolyte leakage testing are preferably determined randomly to remove bias, for example by allocating each rhizome a number and using a random number generator to select a proportion of the rhizomes for testing.

Preferably, the total number of roots and/or rhizomes collected per site and analysed by electrolyte leakage testing is between about 10 to about 100, between about 20 to about 60, between about 30 to about 40, or more preferably between about 30 to about 35 roots and/or rhizomes per site. For example, if three trial pits are excavated as described herein, an average of 10-12 of roots and/or rhizomes are obtained per trial pit. Preferably, each trial pit comprises 10 or less excavation units, 8 or less excavation units, 5 or less excavation units, or 3 or less excavation units (for example excavation units having individual volumes of 1 m$^3$). Preferably, three trial pits comprising 4 excavation units, each excavation unit having a volume of 1 m$^3$, are excavated from the site. Preferably, at least three roots and/or rhizomes, at least six roots and/or rhizomes or at least ten roots and/or rhizomes are randomly selected from each excavation unit analysed by electrolyte leakage testing according to the methods disclosed herein.

The location of trial pits at a site may be randomly allocated or may be determined based on the expertise of a surveyor. For example, if it was known that a particular area of a site was difficult to access or more difficult to treat with a lethal treatment, it may be that at least one trial pit is located in that area. When a site has localised "sub-populations" of a particular plant species as described herein, such as Japanese knotweed, the location of each sub-population may define the location of each trial pit such that a trial pit is located to cover at least a proportion of each sub-population. It may be that only a sub-population covering an area of a certain size is selected as a location for a trial pit, for example a sub-population covering an area of at least 4m$^2$, at least 6 m$^2$ or at least 10 m$^2$.

Preferably, rhizomes that are uncovered from excavation sites and are identified as rotten by visual inspection (and are therefore non-viable or dead) are excluded from electrolyte leakage testing and the CEL$_{sample}$ value of those rhizomes is not determined. It may be unnecessary to analyse by electrolyte leakage testing rhizomes that are assessed as rotten by visual inspection as it can be fairly concluded by visual inspection alone that these samples are dead or non-viable. It may be that the total number of rhizomes collected from an excavation site or sites that are assessed to be rotten by visual inspection is determined and combined with the results of the electrolyte leakage testing of non-rotten samples to draw a conclusion as to the overall viability of a plant population.

Determining the effectiveness of a treatment through sample based analysis

It is desirable to obtain an accurate and reliable assessment of the effectiveness of a lethal treatment applied to a plant or group of plants through analysis of a proportion of the plant material available only. For example, only a proportion of root and/or rhizome material available at a site may be analysed. Methods of selecting a proportion of plant material for analysis are described throughout.

Analysing a proportion of the plant material available may be referred to as a sample based analysis. Preferably, a sample based analysis requires a proportion of the total rhizome and/or root material available at a site be collected from the site according to the methods presented herein (such as collecting plant material from excavation units or trial pits). Samples may then be obtained from collected root and/or rhizome material for electrolyte leakage testing. Additionally or alternatively, a sample based analysis requires a proportion of the root and/or rhizome material collected from a site to be analysed by electrolyte leakage testing methods disclosed herein. For example, samples are taken from a proportion of roots and/or rhizomes collected. Thus, only a proportion of available plant material at a site is used to determine a mean CEL$_{sample}$ value for a given plant population present at that site. A sample based analysis is more time and cost efficient than a population based analysis in which all plant material, for example all rhizomes, recoverable from a site are analysed.

When analysing only a proportion of samples obtainable from a site, it is important that there is sufficient confidence that the mean CEL$_{sample}$ value calculated from that proportion of samples is indicative of the status of the overall plant population. For example, a reasonable certainty is needed that the sampling size was large enough to have identified any viable plant material present at the site e.g. roots and/or rhizomes having a CEL$_{sample}$ value of less than C1. In order to have confidence in a sample based analysis the sampling accuracy may be defined using two parameters; an Acceptable Outcome Level (AOL) and an Unacceptable Outcome Level (UOL). An Acceptable Outcome (AO) is defined as an outcome where it is likely that fewer than v(a)% of rhizomes present at a site have a CEL$_{sample}$ value of less than C1. Here, v(a) defines the AOL. An Outcome (Acceptable or Unacceptable) is likely if the probability of achieving that outcome is at least 80%, at least 90%, at least 95% or at least 99.5%. Preferably, v(a)% is defined as <1/R, wherein R is the estimated number of rhizomes within a population at a given site. Preferably, v(a)% is <1%, <0.1%, or more preferably <0.01%. As an example, when a site contains 10,000 rhizome and v(a) is <0.01%, an acceptable outcome is reached when it is likely that less than 1 of the rhizomes has a $CEL_{sample}$ value of less than C1 (i.e. is viable). An Unacceptable Outcome is defined as an outcome where it is likely that more than v(u)% of rhizomes present at a site have a $CEL_{sample}$ value of less than C1. Here, v(u) defines the UOL. Preferably, v(u)% is chosen according to the level of risk deemed acceptable in falsely identifying a population as dead, when it is in fact still alive, or at least some plants or plant material within the population is still alive (a false negative result). The greater the value of v(u), the greater the risk of incorrectly identifying a plant population as dead. Preferably, v(u)% is >0.1%, >0.5%, >1.0%. >2%, >5% or >10%. More preferably, v(u)% is > 1.0%., i.e. the outcome is unacceptable if it is likely that more than 1% of rhizomes have a $CEL_{sample}$ value of less than C1 (are viable). As an example, when a site contains 10,000 rhizomes and v(u) is >1%, an unacceptable outcome is reached when more than 100 of the rhizomes have a $CEL_{sample}$ value of less than C1 (i.e. is viable).

Thus, the methods disclosed herein may further comprise determining whether the mean $CEL_{sample}$ value of collected samples is indicative that an Acceptable Outcome has been reached, and/or determining whether the mean $CEL_{sample}$ value is indicative that an Unacceptable Outcome has been reached, wherein an Acceptable Outcome is such that fewer than v(a)% of rhizomes of the plant population at a given site are likely to have a $CEL_{sample}$ value of less than C1, and wherein an Unacceptable Outcome is such that more than v(u)% of rhizomes of the plant population at a given site are likely to have a $CEL_{sample}$ value of less than C1. Preferably, v(a)% is <0.01% and/or v(u)% is > 1.0%. The mean $CEL_{sample}$ value is calculated according to any method described herein. Preferably, the mean $CEL_{sample}$ value is calculated from a plurality of samples obtainable from a plant or group of plants at a site according to the methods disclosed herein.

When the mean $CEL_{sample}$ value is indicative that the Acceptable Outcome has been reached, there is a high degree of confidence that the plant population being sampled is dead. When the mean $CEL_{sample}$ value is indicative that the Acceptable Outcome has not been reached, but when the Unacceptable Outcome has also not been reached (i.e. the % of viable rhizomes is between v(a) and v(u)), there may be greater uncertainty as to whether the population of plants is dead or alive, but that level of uncertainty may still be acceptable. When the mean $CEL_{sample}$ value is indicative that the Unacceptable Outcome has been reached, there is an unacceptable level of uncertainty as to whether the general plant population is dead. When the mean $CEL_{sample}$ value is indicative that the % of viable rhizomes is between v(a) and v(u), and/or is greater than v(u), it may be that more samples need to be taken from a site and evaluated by the methods disclosed herein to be able to determine the effectiveness of a lethal plant treatment with an acceptable amount of certainty. This may involve obtaining more root and/or rhizome material from a site, and/or obtaining further samples (i.e. sections) of root and/or rhizome from the material already collected for testing by electrolyte leakage testing. Methods of obtaining sections of root and/or rhizome material for electrolyte leakage testing are described herein. Additionally or alternatively, it may be that when mean $CEL_{sample}$ value is indicative that the % of viable rhizomes is between v(a) and v(u), and/or is greater than v(u), the site requires further lethal treatment as there is an unacceptable level of uncertainty as to whether all plants within the population are already dead.

If a $CEL_{sample}$ value measured for one or more samples obtained from a site is found to be less than C1, then it is assumed that the mean $CEL_{sample}$ value will be above the UOL and the population is determined to be viable or alive. Thus, further treatment of the site is necessary.

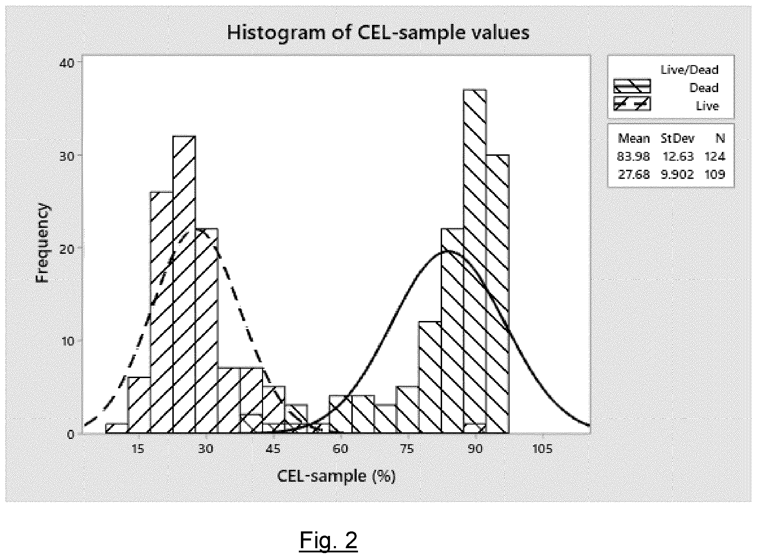

A viability map may be used as a convenient way of interpreting whether mean $CEL_{sample}$ values determined from a sample based analysis meet the Acceptable and/or Unacceptable Outcome. An example of a viability map is shown in Figure 11. Referring to Figure 11, a viability map may take the form of a graphical plot, wherein the x-axis is the mean $CEL_{sample}$ value of a group of samples selected according to any method disclosed herein, and the y-axis is the standard deviation of $CEL_{sample}$ values of the samples. A skilled person will understand that the mean $CEL_{sample}$ values may be represented as a percentage value or a value between 0 and 1, and that both representations are equivalent and interchangeable, for example herein. Point (4) of Figure 11 represents a mean $CEL_{sample}$ value calculated from a group of samples obtained from a site according to the methods disclosed herein. The vertical line (1) shown in Figure 11 represents C1. C1 may have any suitable value disclosed herein, for example C1 may have a value of 0.56. The C1 value may be adjusted according to sample size, plant species, growing conditions, time of year, site location or other variables as described herein. For illustrative purposes, Figure 11 represents C1 as 0.56 (or 56%) and is equivalent to a mean $CEL_{sample}$ value of 0.56 (or 56%). C1 may alternatively be referred to as the rhizome viability threshold. The value of C1 was obtained by selecting the $CEL_{sample}$ value which differentiated between live and dead reference samples as shown in Figure 2 (i.e. C1 was selected as a value of $CEL_{sample}$ wherein all live reference samples had a $CEL_{sample}$ value exceeding C1 and all dead reference samples had a $CEL_{sample}$ value of less than C1). As disclosed herein, when the $CEL_{sample}$ value is less than C1, the rhizome is viable (alive) and when a $CEL_{sample}$ value is greater than C1, the rhizome is not viable (dead). The dashed ellipse (6) of Figure 11 represents the maximum standard deviation at a given mean $CEL_{sample}$ value. The standard deviation may be calculated according to standard mathematical methods well known to the skilled person. The upper curve (2) represents the UOL. A mean $CEL_{sample}$ value lying on or above this curve means that the Unacceptable Outcome has been reached (area (a) of Figure 11) and at least some of the rhizomes obtained from plants within a population are likely to be viable (alive). Preferably, if the mean $CEL_{sample}$ value lies on or above curve (2), the site is treated further with a lethal treatment. In Figure 11, v(u)>1% but this value may be adjusted to any appropriate v(u) value as described herein. The lower curve (3) represents the AOL. A mean $CEL_{sample}$ value lying on or below this curve means that the Acceptable Outcome has been reached (area (c) of Figure 11) and it is likely that all rhizomes of the plant population are non-viable (dead). In Figure 11, v(a) <0.01% but this value may be adjusted to any appropriate v(a) value as described herein. A mean $CEL_{sample}$ value lying between curves (2) and (3) (area (b) of Figure 11) means that neither the Acceptable or Unacceptable Outcome has been reached. Within this region, the amount of uncertainty as to whether the rhizomes of a plant population are viable or non-viable may be acceptable. Preferably, if the mean $CEL_{sample}$ value lies between curves (2) and (3), then additional samples are taken from the site, analysed and a new mean $CEL_{samples}$ value is calculated. This may involve obtaining more root and/or rhizome material from a site, and/or obtaining further samples (i.e. sections) of root and/or rhizome from the material already collected for testing by electrolyte leakage testing. Methods of obtaining sections of root and/or rhizome material for electrolyte leakage testing are described herein. Preferably, if the mean $CEL_{sample}$ value lies between curves (2) and (3) the site is treated further with a lethal treatment.

Curves (1) and (2) were calculated by fitting $CEL_{sample}$ values of dead reference samples to a beta distribution. Methods of obtaining $CEL_{sample}$ values of dead reference samples are disclosed herein. The skilled person will be able to fit the $CEL_{sample}$ values of dead reference samples to the most appropriate distribution curve (which may be other than a beta distribution) depending on the reference samples collected and by using standard mathematical methods known in the art. Without wishing to be bound by theory, it may be that $CEL_{sample}$ values of dead reference samples obtained from any plant species are expected to follow a beta distribution because electrolyte leakage behaviour is expected to be broadly similar across all plant species, or at least the plant species contemplated herein, because of the analogous cell wall/membrane structure of those species. It may be that the most appropriate distribution curve to fit the $CEL_{sample}$ values of dead reference samples for each group of reference samples needs to be determined. Preferably, the $CEL_{sample}$ values of dead reference samples of rhizomes follow a beta distribution when the plant species is Japanese knotweed.

Preferably, the probability that the mean $CEL_{sample}$ value is indicative of the Acceptable Outcome or Unacceptable Outcome having been met is determined. Preferably, a confidence ellipse is calculated wherein the confidence ellipse defines the probability of the mean $CEL_{sample}$ value meeting the Acceptable Outcome or Unacceptable Outcome. Preferably, the confidence ellipse is plotted onto a viability map, wherein the confidence ellipse defines the probability of the mean $CEL_{sample}$ value falling within a certain region of the viability map. Thus, the probability that a mean $CEL_{sample}$ value has met the criteria for AOL or UOL may be defined. The confidence ellipse may be defined as the confidence percentage, c%, that a mean $CEL_{sample}$ value meets the criteria for either AOL or UOL. For example, the confidence level, c%, may be at least 90%, at least 95%, or at least 99.5%. More preferably, the confidence level is 95%. For example, when the confidence level is 95%, the confidence ellipse defines a region of the viability map where there is a 95% confidence that the mean $CEL_{sample}$ value will fall within that region. The area defined by the confidence ellipse is influenced by the sample size used to determine the mean $CEL_{sample}$ value. Referring again to Figure 11, a 95% confidence ellipse (5) was plotted around the mean $CEL_{sample}$ value (4). A confidence ellipse may be determined by a person skilled in the art using standard mathematical methods. For any given mean $CEL_{sample}$ value having a known standard deviation, the probability that the true mean $CEL_{sample}$ value and standard deviation lies within the confidence ellipse (5) is 95%. Thus, according to Figure 11, when the sample size is 36, the mean $CEL_{sample}$ value is 0.80 and the standard deviation is 0.080, then there is a 95% probability that the true mean and standard deviation values lie within the ellipse (5) and therefore intersecting the UOL line (2). In this scenario, there is an unacceptable likelihood that at least some of the rhizomes at the site are viable. Therefore, a further lethal treatment will need to be applied to the plants.

Because the size of the confidence ellipse is influenced by sample size, an appropriate sample size may be determined as one which results in the 95% confidence ellipse falling between the AOL and UOL. Choosing an appropriate sample size in such a manner lessens the risk that a false positive conclusion (the conclusion that viable knotweed is present when it is in fact not) or a false negative conclusion (conclusion that no viable plant is present, when it is in fact present) will be drawn. Although neither a false positive or false negative conclusion is desirable, a false negative is especially undesirable because this may result in the plant species re-populating a site. On the other hand, a false positive result results in additional treatment of the site when there was in fact no viable plants present and the treatment is unnecessary. It may be that obtaining more samples lessens the area of the viability map falling within the confidence ellipse.

When collected roots and/or rhizomes are identified as rotten by visual inspection (that is falling into group 1 as described herein), it is concluded that these samples have 100% probability that they meet the AOL criterion i.e. the probability that the group 1 samples have a $CEL_{sample}$ value of less than C1 is zero. It may be that a weighted average of the calculated probabilities of the mean $CEL_{sample}$ value meeting an AOL or UOL and the assumed probabilities of the visual samples meeting an AOL or UOL is calculated to give the overall probability that the mean $CEL_{sample}$ value meets an AOL or UOL. Preferably, the average probability is weighted according to the number of candidate rhizomes extracted e.g. if the number of group 2 samples collected was 200 and the number of group 1 samples collected is 25, then there is a weighting of 8:1.

Methods of optimising a heat treatment

Specifically, the ELT methods of the present invention may be used to optimise a heat treatment which is applied either to one or more plants or to a sample of root or rhizome of a plant. The method of optimising a heat treatment is a specific mode of validating a treatment, wherein the treatment is a heat treatment.

This method may be particularly advantageous for use on control plots where plants known to be alive are treated under controlled conditions. Alternatively, samples of root or rhizomes may be obtained from a plant and then each sample is exposed to different temperature and/or time conditions to determine the optimum conditions which should be applied to a plant in order to kill it in the most efficient way. This method may be ideally suited to laboratory applications.

The ELT methods of the present invention may be used to determine the $CEL_{sample}$ value of a sample which has been exposed directly to a heat treatment. A different heat treatment, for example at a different temperature, or for a different length of time, is then applied to a different sample. A comparison of the $CEL_{sample}$ values of the first sample and other sample will determine the effectiveness of the heat treatments applied.

The sample to be heat treated may be buried together with at least one temperature probe so that the temperature to which the sample is treated, and the length of time that the sample is heated at that temperature, can be monitored.

Alternatively, the roots or rhizomes obtained from a plant may be buried and heat applied to the surrounding soil in which the plants are buried. A temperature probe may also be buried adjacent to the buried roots or rhizomes, in a location suitable to determine the temperature at which the roots or rhizomes are heated, for example within 50 cm of the plants to be buried. For example, the roots or rhizomes may be tagged so that they are identifiable, and buried together with temperature probes inside a sealed container, such as well casings or net bags. A heat treatment may then be applied to the roots or rhizomes. Because the temperature probe monitors the temperature applied to the root or rhizome sample, the viability change of the sample can be directly linked to the temperature and time length of exposure to the sample.

$CEL_{sample}$ of samples of root or rhizome used as controls, to which no treatment is applied, may also be determined.

Examples

Equipment

A Thermo Scientific® Orion Star™ A112 conductivity meter kit (commercially available from Fisher Scientific UK, Loughborough) was used to measure conductivity values. A conductivity calibration solution was used to calibrate the conductivity meter; for example Eutech® EC-CON-1413BT calibration solution (Eutech Instruments Pte Ltd, Singapore). Purified water was obtained from a suitable reverse osmosis laboratory supply. The minimum standard of purified water (also referred to as deionised water or distilled water) is ISO grade 2 (conductivity 1.0 $\mu$S/cm at 25 °C), and ISO grade 1 (conductivity 0.1 $\mu$S/cm at 25 °C) is preferred.

28-mL Universal glass vials (sometimes identified as "McCartney" vials), with wide mouth and plastic screw cap were used for containing samples. The vials were held in racks. Further equipment used includes: a hot water bath set to 90 °C, a pipette or other dispenser for dispensing 15 mL of solution, tweezers sized to fit within a vial, scalpel and blades, junior size hacksaw with fine tooth blade (150 mm), a high density fibreboard for resting samples on for cutting, buckets for wash water (5 x 10 L), paper tissue for drying samples, laboratory alkaline detergent, and a waste bin with liner for safe storage of Japanese knotweed offcuts to be disposed of by autoclave, incineration or other method approved for disposal of Japanese knotweed.

Sample preparation and ELT analysis were carried out in an enclosed work space, maintained at room temperature (15-20 °C). A laboratory bench space of at least 2 m plus additional space for sample washing buckets and waste containers was used. Where applicable, equipment was cleaned before use and dried in a warm space or drying oven. A box or other dark space was used for storing samples overnight, before and during analysis.

Field sampling, selection and preliminary preparation of sample rhizome

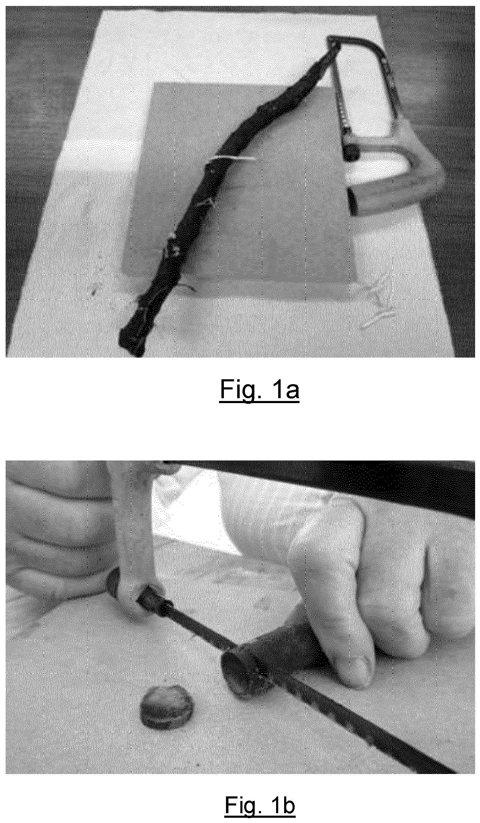

A Japanese knotweed crown was excavated from a site where Japanese knotweed was present. At least three rhizome lengths were cut from the parent crown. Rhizomes were selected to give a representative sample of the parent plant. The rhizomes selected were without major physical damage and were approximately cylindrical and between 10 mm and 30 mm in diameter and at least 100 mm in length. Figure 1a shows a typical Japanese knotweed rhizome selected for analysis. If a propagation method is also to be used, the rhizome lengths must include nodes. Soil adhering to the rhizomes was removed by gently shaking the rhizomes. A thorough cleaning of the rhizomes was carried out in the laboratory as detailed below.

## Preparation of sample material

Rhizome material was prepared according to the following general procedure.

A sample of rhizome material was obtained by cutting a rhizome from the parent crown, as described above. Soil covering the rhizome was brushed off, taking care not to damage the bark. The rhizome was submerged consecutively in purified water held in three buckets. Each time, while the rhizome was submerged in purified water, the surface of the rhizome was gently rubbed with fingers to remove soil and debris. At this stage, the rhizome should not be left to stand in water or soaked to prevent electrolyte (solutes) leakage from the rhizome. Following rinsing, the rhizome was gently blotted with tissue paper to remove excess water. A scalpel was used to removed fine rootlets growing from the rhizome. A 2-3 mm section of the ends of the rhizome were removed to remove any potentially dead or damaged material.

## Rhizome sample preparation

The selected rhizome was a lignified rhizome with a diameter of between about 10 and 30 mm. Sample slices of 2 mm in thickness (which typically equates to a weight of about 100 to 500 mg) were cut from the rhizome, using a scalpel or hacksaw, depending on the size and toughness of the rhizome. A piece of fibreboard or wood may be placed underneath the rhizome before cutting to provide a stable base on which to cut (Figures 1a and 1b). Each sample may be further cut, for example in half to form two generally semi-circular disc portions, in order to fit the sample into a suitable vial; for example a Universal type glass vial of volume 28 mL with a screw cap. Only one of the cut portions of the slice is then used. When cutting the slices, nodes, blemished areas of bark and damaged areas should be avoided.

After cutting to size, the portion of sample was very briefly submerged in deionised water to remove surface contamination and then gently blotted dry with tissue paper. Each sample was then placed in a separate vial. The precise size and shape of the samples is not critical but the thickness should conveniently be between 1 and 3 mm, to remove potential variations in diameter caused by differences in sample size. The sample must be fully submerged after deionised water is added to the vial.

15 ± 2 mL of deionised water was added to the vials containing the samples. The exact volume of water is not critical, but enough water must be added to cover the sample as well as a conductivity probe, when it is inserted into the vial. The cap is then put on the vial and the vial is gently inverted twice and then stored in a dark room at room temperature (about 15-20 °C) overnight for 16 hours ± 30 minutes. Multiple samples may be prepared in separate vials. For each batch of samples to be analysed, three blanks were prepared with only purified water in the vial.

## Method for measuring $CEL_{sample}$ - Electrolyte leakage testing (ELT)

The conductivity meter was calibrated using a conductivity standard solution, such as the Eutech® EC-CON-1413BT calibration solution mentioned above, according to known methods in the art. Each vial containing a sample was then gently inverted twice. The sample was then removed from the vial using tweezers before inserting the conductivity probe into the solution. The sample was stored so that it could be returned to the vial immediately after a conductivity reading had been taken. The meter probe was placed in the sample water and the conductivity measured to give the value $EL_{sample}$. The sample was then returned to the vial and the cap replaced firmly. The meter probe and tweezers were rinsed with purified water and the tweezers dried.

The vial containing the sample was then placed in a water bath at 90 °C for 30 minutes. The water level in the bath must be above the level of water inside the vials. The vial was then be cooled to room temperature. To speed up this step, the sample vial may be placed in a cold water bath. Ideally the conductivity measurements are carried out on the sample before and after heat treatment at the same temperature, e.g. at room temperature.

The vial was then gently inverted twice and the sample was then removed from the vial with tweezers. The conductivity of the solution was measured again to give the value $EL_{total}$.

The conductivity probe should be regularly checked with the calibration standard solution. The conductivity of the blank samples were also measured.

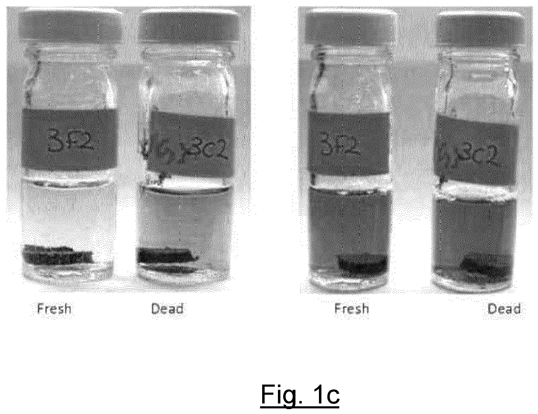

Figure 1c shows an image of fresh (live) and dead lignified rhizomes from a Japanese knotweed plant in Universal vials and deionised water during ELT measurement. The image on the left shows the samples after the overnight incubation period (16 hours) and the image on the right shows the samples after being heated at 90 °C for 20 minutes.

Calculations

The mean conductivity of the three blanks was calculated. This was subtracted from each conductivity reading of the samples, before and after heat treatment. The blank-corrected conductivity measurement before heating is referred to as $EL_{sample}$ and the blank-corrected conductivity after heating is referred to as $EL_{total}$ .
The comparative electrolyte leakage (CEL) was then calculated for each sample as follows:

$$CEL_{sample} = 100 \times \left(\frac{EL_{sample}}{EL_{total}}\right)$$

Disposal of samples

The analysed sample is dead following heat treatment. The samples should be disposed of appropriately. The sample vials should be washed with a laboratory alkaline detergent, rinsed with deionised water and dried before further use.

Derivation of critical or threshold values for interpretation of the ELT results

In order for the results, i.e. the $CEL_{sample}$ values of the samples, to be interpreted, it is necessary to identify suitable "critical" or "threshold" values in order to establish whether a sample is dead or alive. In the present example, the critical or threshold values were derived from a range of samples obtained from different sites and when the plants have been exposed to different conditions; for example to take account of seasonal effects.
In particular, rhizomes of Japanese knotweed were obtained from ten locations over the period of September 2015 to July 2016. The samples were obtained from 10 different field sites and were sampled over a period of approximately eight months. As a result, there could possibly be a difference in the measured $CEL_{sample}$ values for the rhizomes obtained from the different sites, owing to variability resulting from genetic variation, environmental conditions, sampling techniques and/or sample storage time. A number of samples, both alive and dead, were therefore analysed to determine critical values with at least 95% confidence. The aim was to derive critical values C1 and C2, as described above. In the laboratory, dead samples were prepared from the live material, for example by heating the samples at 50 °C for 2 hours. In the present example, the results provided 109 $CEL_{sample}$ values for live samples and 124 $CEL_{sample}$ values for dead samples. It will be understood that in different embodiments, more or fewer live and/or dead samples may be used, provided the derived critical value C1 allows identification of dead test samples with a confidence of at least 95%, and, in some embodiments, C2 further allows identification of live test samples with a confidence of at least 95%.
Figure 2 shows a histogram of all $CEL_{sample}$ values (%), with live and dead samples identified, showing fitted normal distributions. A clear separation between the mean $CEL_{sample}$ values for live and dead samples is visible. However, it can be seen that in this example, there is a small region of overlap in the distributions where both live and dead samples gave similar CEL values. Samples falling within the intermediate region may be reanalysed in order to try and reach a definitive conclusion.
$CEL_{sample}$ values (%) of live and dead classes of sample were assessed separately. Both failed normality tests, being significantly skewed. The transformations $log_{10}CEL$ and $log_{10}(100-CEL)$ were applied to live and dead values respectively. The distribution for dead samples was still not normal, but quite close to normality.

| | Mean | Mean +2SD | Mean -2SD | | Quartile 1 | Median | Quartile 3 |
|---|---|---|---|---|---|---|---|
| Live | 26.4 | 48.4 | | | 21.9 | 26.2 | 30.5 |
| Dead | 87.4 | | 48.6 | | 80.5 | 88.3 | 92.3 |

Table 1. $CEL_{sample}$ (%) summary statistics overall results

Half of the samples yielded $CEL_{sample}$ values within quite narrow ranges: 50% of live samples had $CEL_{sample}$ values between 21.9% and 30.5% (Quartiles 1 and 3), and 50% of dead samples had CEL values between 80.5% and 92.3% (Quartiles 1 and 3) (Table 1).
In a normal distribution, 95% of samples lie between ±2SD of the mean. In this example, 97.5% of live samples should have CEL <48.4%, while 97.5% of dead samples should have CEL >48.6% (Table 1). Thus, CEL=48.5% is a potential critical value (C1) which discriminates between live and dead samples to about 95% accuracy or more.
Using the single critical value (C1) of 48.5% for this dataset, seven samples (3.0% of the total) were incorrectly identified. As described above, in some embodiments it may be beneficial to specify an "overlap" or "intermediate" range for inconclusive, borderline results where reanalysis or resampling/reanalysis, either by ELT or a complementary method, may be used. Of the samples analysed, an obvious extreme $CEL_{sample}$ value of 89% for one live sample was considered to be an outlier and discarded. The remainder of the samples which were incorrectly identified under a 48.5% critical value (C1) fell into the histogram columns 37.5% to 57.5% (Figure 2); a total of 21 samples (9% of the total) fell into this range. In accordance with the present invention, the outer limits of these columns were used to define critical values such that an even higher degree of accuracy of identifying dead samples could be obtained, if required. The upper limit was determined to be 57.5% which is a suitable value for C1 when a higher degree of accuracy is required, and the lower limit was determined to be 37.5% which is defined as C2..

Of the 21 samples in the "overlap" or "intermediate" range, 14 were actually live and 11 were from the same site (Site 4). Seven of those 11 samples, plus the extreme outlier (Live, 89%), came from a batch of rhizomes which had been stored for six weeks in a lab before cutting for the experiment. This was the longest delay between obtaining a rhizome from a plant and preparing samples for analysis. Without being bound by theory, it is suggested that the delay between sampling and analysis may have been responsible for the inconclusive results for these samples. Delay between sampling from the field location and analysis may increase variability and reduce the accuracy of the ELT method. It may therefore be desirable to prepare and analyse samples of the rhizome within a short time scale to improve the accuracy of the methods of the invention, i.e. within about 72 hours, and preferably within about 48 hours.

Statistical analysis of $CEL_{sample}$ values, classified by originating site

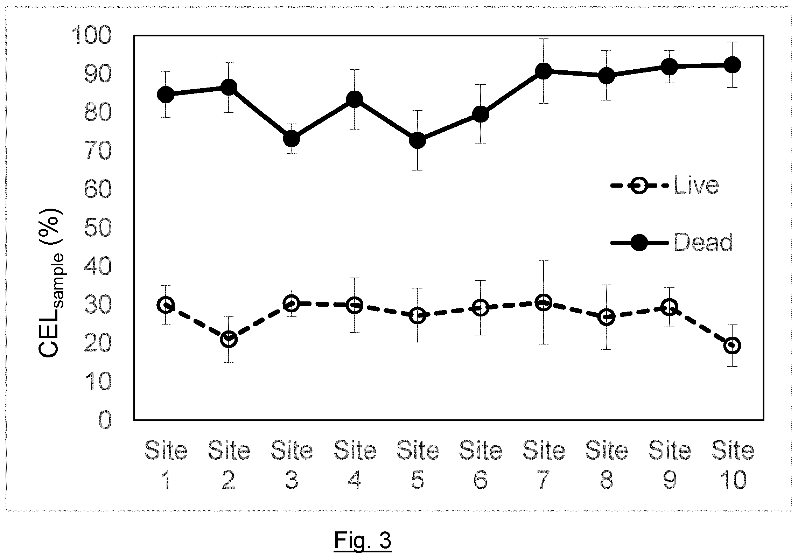

$CEL_{sample}$ values for live and dead samples were analysed by one-way ANOVA with the sampling site as the factor (Figure 3). Possible outliers and "overlap" results were included in the data. Site-specific mean values for live (bottom line) and dead (upper line) material were separated clearly with 95% confidence intervals based on standard errors of the pooled data. Comparisons between sites using the Tukey multiple range test showed that site 10 gave live sample $CEL_{sample}$ values which were significantly lower than those for sites 1, 3 and 9. Meanwhile, sites 3 and 5 gave dead sample $CEL_{sample}$ values which were lower than those for sites 7, 8, 9 and 10. Differences between mean $CEL_{sample}$ values from live and dead samples appeared rather less for sites 3, 4, 5 and 6, and it is thought that this might in part be because the JK samples from those sites were obtained in the autumn or winter (October 2015 or February 2016) when the plants were not in vigorous growth.

The mean site mean $CEL_{sample}$ values were 26.2% and 87.8% for live and dead Japanese knotweed respectively (Table 2). The upper 95% confidence interval for live Japanese knotweed (32.2%) (C2) was below the all-samples single critical value (C) of 48.5%. Similarly, the lower 95% critical value (C1) for dead Japanese knotweed (72.4%) was above 48.5% (C).

| Table 2. Summary statistics for mean $CEL_{sample}$ (%) values for the 10 sites | | | |
|---|---|---|---|
| | Mean | Mean +2SD | Mean -2SD |
| Live | 26.2 | **32.2*** | 21.4 |
| Dead | 87.8 | 94.6 | **72.4*** |

Using the values in Table 2 for C1 and C2, such that C1 is 72.4 and C2 is 32.2, allowed the $CEL_{sample}$ mean from each site to be classified correctly. However, a small proportion of the individual samples fell between C1 and C2. It is therefore desirable that enough samples should be taken from sites or plants to ensure that "false" individual values do not excessively bias the mean result. In the present example, the number of samples contributing to each site mean varied widely, from 3 to 30. The median number was 8.5 for live and 10 for dead.

This aspect was examined using the $CEL_{sample}$ values of the samples obtained from site 6 only. Site 6 was chosen because:

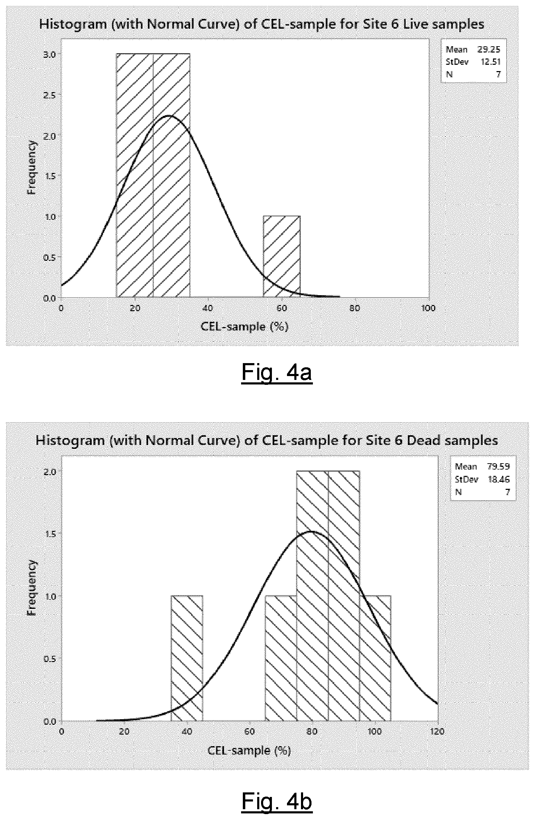

(i) there were seven results for each of live and dead values samples, and this is a reasonable number of replicates to handle if an experimental run has to deal with many sites;
(ii) each of the live and dead groups contained one outlier falling into the "overlap" region of unreliable interpretation (Figs.4a and 4b);
(iii) the alternative scenarios that site 6 might be either live or dead were represented equally in the above conditions.

The results for Site 6 (Figures 4a and 4b) imply that with seven replicate samples, one extreme value, on the "wrong" side of the single all-samples critical value (C), does not excessively bias the mean $CEL_{sample}$ result. More

broadly, no more than 10-15% of the samples were outliers. Therefore, provided that no more than 15% of the samples analysed lie on the "wrong" side of the critical value C, then the status of the samples can be accurately determined.

For practical purposes, one exemplary sampling strategy in accordance with the invention may comprise harvesting three rhizomes per plant, cutting nine sample slices and analysing seven slices obtained from the three rhizomes, first rejecting the two slices that are least perfect in size (e.g. missing bits of bark, or having an irregular shape). It will be understood that this sampling method may be varied depending on what is practical on site. Thus, in some embodiments, at least 5 replicate samples per site may be taken. Such samples be taken from at least three rhizomes from one plant. At least one plant per site should be sampled, although two or more plants or more than three plants may be sampled per site.

This above-described interpretation of the results of analysis of seven samples is summarised below in Table 3, using values which are rounded off from those presented in the discussion above.

| Table 3. Conditions for interpretation of $CEL_{sample}$ values (%) from seven analysis samples from rhizome of a JK plant. | | | |
|---|---|---|---|
| Interpretation | CEL value for the mean of 7 analysis samples /% | | Maximum of one analysis sample only allowed with this $CEL_{sample}$ value |
| Live | < 32 | AND | >50 |
| Dead | >72 | AND | <50 |
| Result inconclusive | Results do not meet both of the conditions specified | | |

In summary, ELT in accordance with the invention is a robust analysis method which can be used to determine whether a sample is alive or dead with over 95% accuracy, and it can be used to determine whether a lethal treatment of an invasive plant has been successful. Meaningful CEL values can be obtained from multiple plants across multiple sites, and collected under different conditions to give critical values which inform the analysis and result in highly accurate determination of the status of a sample. Analysis can be carried out in as little as 1.5 days and is easy and inexpensive to perform.

## Complementary testing methods

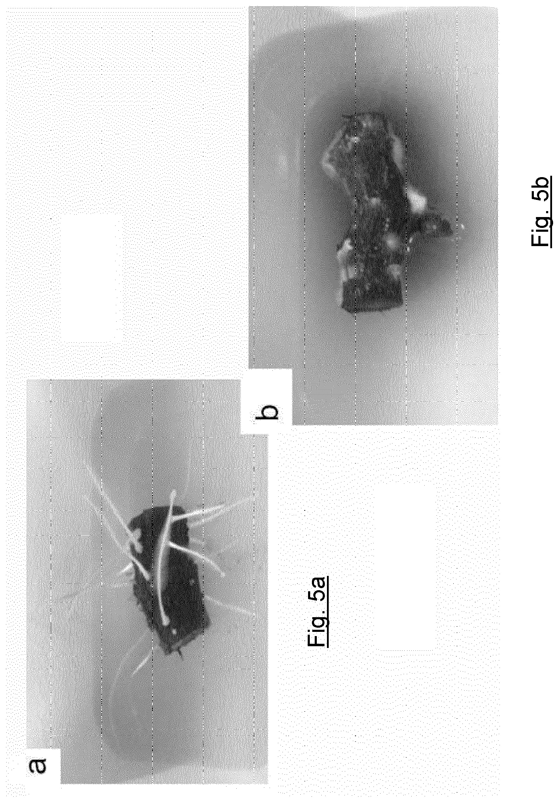

A simple but slower method may be used to test the ability of a section of rhizome to regrow in a propagation test. This method can be used to help confirm whether a sample is dead or alive, particularly if a sample falls into the intermediate range discussed above, although complementary testing can be used with all samples. A piece of rhizome that includes a root node may be incubated on agar (or another suitable growth medium) to test the ability of the sample to regrow. If a sample is alive then root hairs will grow from the rhizome (see Figure 5a), whereas if the sample is dead, no growth will be observed (Figure 5b). It can be seen in Figure 5b that decay has started in the dead sample, shown by fungal growth and leakage of dark contents of the dead sample into the agar. The growth of a sample on agar provides an accelerated result over re-germination of the sample in compost.

## Example 1: Lethal treatment method

The following method describes an example of how ELT can be applied to determine the effectiveness of a lethal treatment in accordance with the present invention.

One or more plants that are growing on a site where they are not wanted are treated to kill the one or more plants. A suitable method of lethal treatment is a heat treatment. Such a heat treatment may be applied under the soil surface to a depth of around 2 m. Ideally, the soil is heated at 50 °C for 2 hours in order to have the best chance of killing the one or more plants. In this way, the root or rhizome system of the plant is targeted.

After the treatment is finished a sample of root or rhizome is taken for ELT, according to the methods described previously herein. ELT can be used to determine whether or not the treatment applied has been effective to at least 95% accuracy.

In this treatment configuration, either the same person may treat and test the samples by ELT or different parties may carry out the treating and ELT work. This treatment method can be used for example to determine whether a treatment carried out by a third party has been effective, or if further treatment is required.

Example 2: Optimisation of temperature treatments

ELT can also be used in accordance with the invention to determine the optimum time and/or temperature for killing a plant, for example Japanese knotweed. Applying a heat treatment to a plant for longer than necessary is inefficient and expensive. Likewise, the application of unnecessarily high temperatures should be avoided if a cost effective treatment is desired.

The optimum time or temperature at which a heat treatment is applied to a plant can be determined as follows.

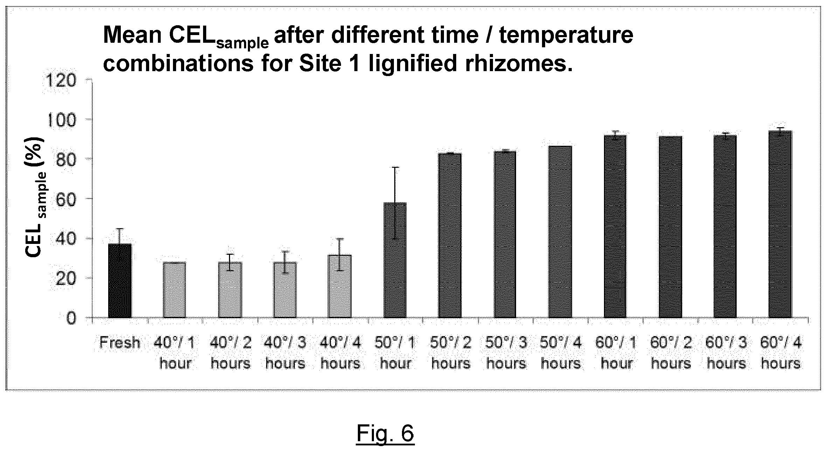

Samples were obtained from Japanese knotweed plants as described above and $CEL_{sample}$ value determined, also as described above. As shown in Figure 6, samples of lignified rhizome were heated at a range of different respective temperatures from 40 °C to 60 °C for between 1 and 4 hours and the $CEL_{sample}$ value of the samples determined after the heat treatment. Each individual experiment was replicated at least once. Temperatures of 80 °C or higher were excluded as the sample structure would be damaged at such elevated temperatures and ELT could not then be carried out. Heating the sample at 2 hours at 50 °C was found to be sufficient to kill a sample and was sufficient to create a significant difference (>2 SD) between it and less vigorous treatments.

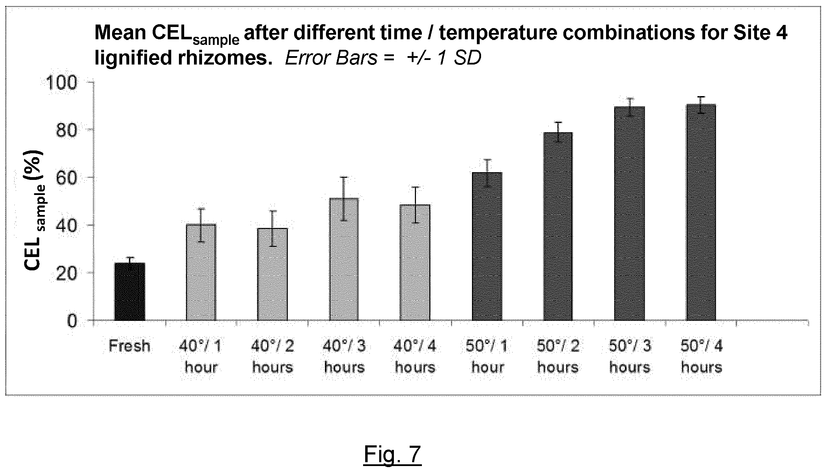

As shown in Figure 7, the above-described heat and time experiments were repeated but this time with more replication, with each time and temperature experiment being carried out ten times. Heating the samples at 50 °C for 2 hours was sufficient to kill the samples when assessed by ELT. A heat treatment of 50 °C for 2 hours is therefore considered sufficient to kill Japanese knotweed rhizomes. However, in some embodiments, the samples may be treated at a higher temperature, e.g. at 60 °C, and/or for longer.

Similar methods may be applied to root or rhizome systems of other plant species to determine optimum heat treatments for killing the plant. These methods may also be used to determine the effectiveness of other treatments, for example biological, chemical, mechanical or physical treatments. The methods may also allow a site specific treatment to be developed whereby samples of a plant to be treated are taken, treatment under various conditions is applied to the samples, and then ELT is used as described above to determine which treatment is most efficient to apply to a particular site.

Example 3: Comparative testing methods

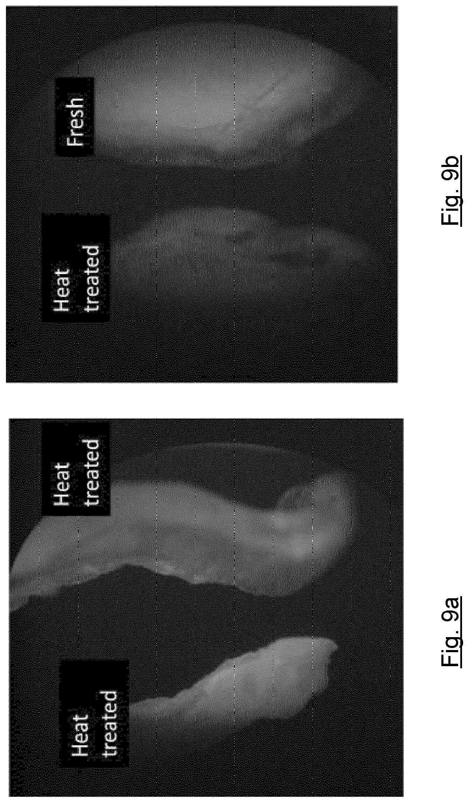

Existing qualitative staining techniques are ineffective for testing lignified rhizomes of Japanese knotweed to verify whether the rhizomes are dead or still alive. This is at least in part because lignified rhizomes contain lignin which reacts with the key vital stain used in staining techniques, i.e. 2,3,5-triphenyltetrazolium chloride (TTC). Another reason is because visual evaluation of the vital stain, fluorescein diacetate, is laborious and open to error. As shown in Figure 9, samples which have been heat treated may have a wide variety of different appearances, so it is difficult to draw any conclusions from images alone.

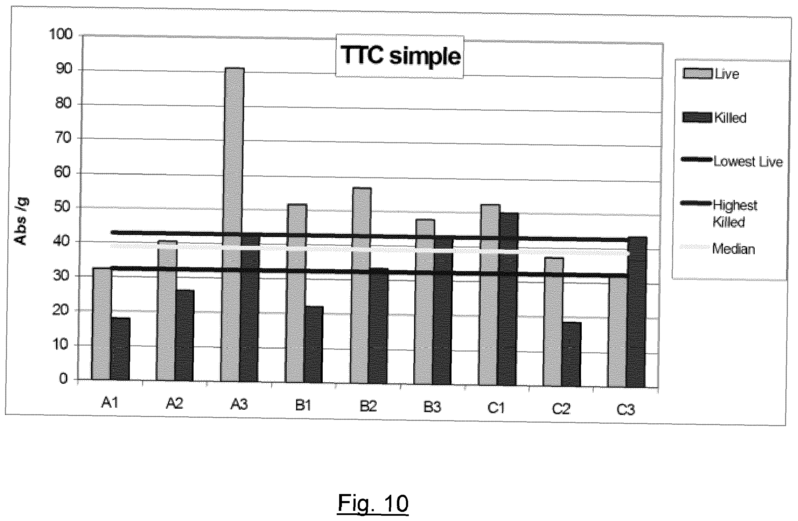

Colorimetery is also unsuccessful in quantitatively analysing rhizome samples to determine if they were alive or dead. Samples were stained with TTC and their absorption measured (see Figure 10). The method was laborious and the number of samples falsely identified as live or dead was unacceptably high for use as a practical validation method of the effectiveness of a treatment.

While the present invention has been described and illustrated with reference to particular embodiments, it will be appreciated by those of ordinary skill in the art that the invention lends itself to many different variations not specifically illustrated herein.

Where in the foregoing description, integers or elements are mentioned which have known, obvious or foreseeable equivalents, then such equivalents are herein incorporated as if individually set forth. Reference should be made to the claims for determining the true scope of the present invention, which should be construed so as to encompass any such equivalents. It will also be appreciated by the reader that integers or features of the invention that are described as preferable, advantageous, convenient or the like are optional and do not limit the scope of the independent claims. Moreover, it is to be understood that such optional integers or features, whilst of possible benefit in some embodiments of the invention, may not be desirable, and may therefore be absent, in other embodiments.

Aspects and embodiments of the invention are described by the following numbered clauses.

Clause 1. A method of determining the effectiveness of a lethal treatment applied to a plant or a group of two or more plants of the same plant species or variety; the method comprising assessing whether the treated plant or group of plants is dead on the basis of a comparative electrolyte leakage value ($CEL_{sample}$) of one or more root or rhizome samples taken from the treated plant or group of plants; wherein $CEL_{sample}$ is obtained by:

a) measuring the electrolyte leakage of a root or rhizome sample ($EL_{sample}$);
b) thereafter, exposing the sample to cell barrier disrupting conditions;
c) measuring the electrolyte leakage of the sample ($EL_{total}$) again, after the sample has been exposed to the cell barrier disrupting conditions; and

d) calculating $CEL_{sample}$ as:

$$CEL_{sample} = 100 \times \left(\frac{EL_{sample}}{EL_{total}}\right).$$

Clause 2. The method according to clause 1, which comprises assessing the status of the or each sample individually on the basis of its $CEL_{sample}$ value.

Clause 3. The method according to clause 2, which comprises comparing the $CEL_{sample}$ value with a first critical value (C1) and indicating the sample to be dead when $CEL_{sample}$ is equal to or greater than C1.

Clause 4. The method according to clause 3, which comprises comparing the $CEL_{sample}$ value with a second critical value (C2) and indicating the sample to be alive when $CEL_{sample}$ is less than C2; wherein C2 is less than or equal to C1.

Clause 5. The method according to clause 4, wherein C2 is less than C1, the method comprising indicating the status of the sample to be indeterminate when $CEL_{sample}$ is equal to or greater than C2 and less than C1.

Clause 6. The method according to clause 1, which comprises calculating individual $CEL_{sample}$ values for a plurality of samples of the plant or group of plants; deriving a mean $CEL_{sample}$ value from the individual $CEL_{sample}$ values, and assessing whether the treated plant or group of plants is dead on the basis of the mean $CEL_{sample}$ value.

Clause 7. The method according to clause 6, which comprises comparing the mean $CEL_{sample}$ with a first critical value (C1) and indicating the plant or group of plants to be dead when mean $CEL_{sample}$ is equal to or greater than C1.

Clause 8. The method according to clause 7, which comprises comparing the mean $CEL_{sample}$ value with a second critical value (C2) and indicating the plant or group of plants to be alive when mean $CEL_{sample}$ is less than C2; wherein C2 is less than or equal to C1.

Clause 9. The method according to clause 8, wherein C2 is less than C1, the method comprising indicating the status of the plant or group of plants to be indeterminate when mean $CEL_{sample}$ is equal to or greater than C2 and less than C1.

Clause 10. The method according to clause 5 or clause 9, which further comprises using a complementary method to assess the status of the plant or group of plants; wherein the complementary method is optionally a propagation testing method in which the treated plant or group of plants is assessed to be alive when there is observable biological growth during the propagation testing method from one or more root or rhizome samples taken from the treated plant or group of plants.

Clause 11. The method according to any of clause 3-5 or 7-10, wherein C1 is determined by reference to $CEL_{sample}$ values that have been obtained from a plurality of known dead reference samples taken from rhizomes or roots of the invasive plant species or variety to be treated; the critical value C1 being selected as the $CEL_{sample}$ value that identifies the dead reference samples with at least about 95% accuracy.

Clause 12. The method according to clause 11, wherein the critical value C1 is calculated as mean $CEL_{sample}$ -2SD of the dead reference samples.

Clause 13. The method according to clause 11, wherein the known dead reference samples comprise a plurality of samples originating from each one of a plurality of different sites, and the critical value C1 is calculated as a mean site mean $CEL_{sample}$ -2SD of mean $CEL_{sample}$ values of the dead reference samples from the different sites.

Clause 14. The method according to any of clauses 4, 5 or 8-10, wherein C2 is determined by reference to $CEL_{sample}$ values that have been obtained from a plurality of known live reference samples taken from rhizomes or roots of the same invasive plant species or variety as the treated plant or plants, and the critical value C2 is selected as the $CEL_{sample}$ value that identifies the live reference samples with at least about 95% accuracy.

Clause 15. The method according to clause 14, wherein the critical value C2 is calculated as mean $CEL_{sample}$ +2SD of the live reference samples.

Clause 16. The method according to clause 14, wherein the known live reference samples comprise a plurality of samples originating from each one of a plurality of different sites, and the critical value C2 is calculated as a mean site mean $CEL_{sample}$ +2SD of mean $CEL_{sample}$ values of the live reference samples from the different sites.

Clause 17. The method according to any of clause 11-16, wherein the plant or plants from which the reference samples were obtained were grown under the same conditions as the plant to be treated is growing.

Clause 18. The method according to any of clauses 11-16, wherein the reference samples were obtained from one or more plants at the same time of year as the lethal treatment is applied to the plant to be treated.

Clause 19. The method according to any of clauses 3-5 or 7-13, wherein C1 is a value between about 45% and about 75%; for example about 48.5%, more preferably about 56%. Clause 20. The method according to any of clauses 3-5 or 7-13, wherein C1 is a value between about 55% and about 75%; for example about 57.5%.

Clause 21. The method according to any of clauses 3-5, 7-13, 19 or 20, wherein C1 is a value of at least about 70%; for example a value of about 72%.

Clause 22. The method according to any of clauses 4, 5, 8-10 or 14-16, wherein C2 is a value that is less that C1 and between about 30% and about 60%; e.g. about 37.5%.

Clause 23. The method according to any of clauses 4, 5, 8-10 or 14-16, wherein C2 is a value that is less that C1 and between about 30% and about 40%; for example a value of about 32%.

Clause 24. The method according to any preceding clause, wherein the cell barrier disrupting conditions of step b) comprise exposing the sample to at least one of sonication, ionising (e.g. gamma) ionising radiation, heat treatment, microwave radiation, radiofrequency radiation, physical attrition or a chemical agent (e.g. for altering the pH of an aqueous medium containing the sample); wherein preferably the treatment is a heat treatment; for example wherein the sample is heated to about 90 °C; optionally for at least 20 minutes.

Clause 25. The method according to any preceding clause, wherein the sample is submerged in deionised water before step a).

Clause 26. The method according to any preceding clause, wherein the sample is stored in the dark before step a).

Clause 27. The method according to any preceding clause, wherein the sample is stored at room temperature before step a).

Clause 28. The method according to any preceding clause, wherein the sample is stored overnight; for example for at least 16 hours, before step a).

Clause 29. The method according to any preceding clause, wherein the thickness of the rhizome sample analysed in steps (a-d) is between about 1 mm and about 3 mm; preferably about 2 mm, and/or the sample is obtained from a lignified rhizome having a diameter of between about 10 mm and about 30 mm.

Clause 30. The method according to any preceding clause, wherein the plant is a species selected from Japanese knotweed, Dwarf Japanese knotweed, Giant knotweed, Bohemian knotweed, Himalayan knotweed, railway yard knotweed, Rosa rugosa, rhododendron, buddleja, ferns, horsetails, brambles, nettles, bamboo and any other perennial plant.

Clause 31. The method according to any of clauses 3-5, or clauses 10-30 when dependent on clause 3, further comprising assessing the status of the plant or group of plants before treatment by comparing a $CEL_{sample}$ value of one or more root or rhizome samples taken from the plant or group of plants before treatment against C1 and/or, when dependent on clause 4, C2 .

Clause 32. The method according to any of clauses 7-9, or clauses 10-30 when dependent on clause 7, further comprising assessing the status of the plant or group of two or more plants before treatment by comparing a mean $CEL_{sample}$ value of a plurality of root or rhizome samples taken from the plant or group of plants before treatment against C1 and/or, when dependent on clause 8, C2.

Clause 33. The method according to any one of clause 6-32, wherein the plurality of samples of the plant or group of plants are obtained from roots and/or rhizomes collected from a site where the plant or group of plants are located, wherein the site is divided into multiple unit areas or unit volumes, and wherein the root and/or rhizomes are collected from a proportion of the total unit areas or unit volumes.

Clause 34. The method according to any of clauses 6-33, which comprises determining whether the mean $CEL_{sample}$ value is indicative that an Acceptable Outcome has been reached, and/or determining whether the mean $CEL_{sample}$ value is indicative that an Unacceptable Outcome has been reached, wherein an Acceptable Outcome is such that it is likely that fewer than v(a)% of root or rhizomes of the plant or group of plants have a $CEL_{sample}$ value of less than C1, and wherein an Unacceptable Outcome is such that it is likely that more than v(u)% of root or rhizomes of the plant or group of plants have a $CEL_{sample}$ value of less than C1, preferably wherein v(a)% is <0.01% and/or v(u)% is > 1.0%. Clause 35. A method for the treatment of a plant or group of two or more plants, the method comprising:

  i) applying a lethal treatment to the plant or group of plants; and
  ii) determining the effectiveness of the lethal treatment according to any one of clauses 1 to 34.

Clause 36. The method according to clause 35, wherein the lethal treatment is applied to the root or rhizome system of the plant or group of plants.

Clause 37. The method according to clauses 35 or 36, wherein the lethal treatment comprises one or more of a heat treatment, a herbicide treatment, a treatment with a chemical agent, electrical treatment, a pH treatment and/or a stem injection treatment.

Clause 38. The method according to clause 37, wherein the heat treatment is a heat treatment, such as a steam treatment or, preferably, a hot air treatment, which is carried out *in situ* or *ex situ.*

Clause 39. The method of any of clauses 35-38, wherein the effectiveness of the lethal treatment is determined by burying one or more reference portions of known viability of the same species as the plant or plants to be treated in soil with the plant or plants to be treated and assessing the status of the one or more reference portions after the treatment, and optionally during the treatment.

Clause 40. The method of clause 39, wherein the one or more reference portions comprise whole plants or one or more segments of a root or rhizome system of the same species as the plant or plants to be treated; for example Japanese Knotweed.

Clause 41. The method of clause 39 or clause 40, wherein the reference portions are contained in a retrieval container; optionally with 1-5 reference portions per retrieval container.

Clause 42. The method of any of clauses 39-41, wherein the lethal treatment is carried out *ex situ* and one or more reference portions in one or more retrieval containers are added to the soil prior to and optionally during application of the lethal treatment to the soil.

Clause 43. The method of clause 42, wherein one retrieval container is deployed per 50-150 m$^3$ soil, e.g. per 100 m$^3$.

Clause 44. The method of any of clauses 39-41, wherein the lethal treatment is carried out *in situ* at the site where the unwanted plant or plants are growing, and one or more reference portions in one or more retrieval containers are buried at spaced locations and at different levels.

Clause 45. The method of clause 44, wherein a well casing, for example a metal well casing, is driven into the subsurface at each location, with the one or more retrieval containers placed within the casing at different depths; e.g. one bag per 0.1-1.0 m.

Clause 46. The method of any of clauses 39-45, wherein one or more probes are included in or buried adjacent the one or more retrieval containers.

Clause 47. The method of clause 46, wherein the one or more probes are non-wired probes, loggers and/or temperature probes.

Clause 48. The method of any of clauses 39-47, wherein determining the effectiveness of the lethal treatment further comprises assessing the viability of the one or more plants or groups of plants to be treated which are native to the site where they are unwanted.

Clause 49. A method of validating a treatment for killing a plant having a root or rhizome system, the method comprising;

i) determining a CEL$_{sample}$ value of at least one first sample of the root or rhizome system of a specimen of the plant by:

a) measuring the electrolyte leakage of the first sample (EL$_{sample}$);
b) exposing the first sample to cell barrier disrupting conditions;
c) measuring the electrolyte leakage of the first sample (EL$_{total}$) after the sample has been exposed to the cell barrier disrupting conditions; and
d) calculating the comparative electrolyte leakage of the first sample (CEL$_{sample}$) :

$$CEL_{sample} = 100 \times \left(\frac{EL_{sample}}{EL_{total}}\right)$$

ii) applying a treatment to the specimen;
iii) determining the CEL$_{sample}$ of at least one second sample of the root or rhizome system of the specimen after treatment; and
iv) comparing the CEL$_{sample}$ of the first and second samples before and after the treatment to determine the effectiveness of the treatment applied.

Clause 50. A method for optimising a heat treatment for killing a plant having a root or rhizome system, the method comprising;

i) determining if a portion of the root or rhizome system of a specimen of the plant is viable by:

a) measuring the electrolyte leakage of at least one sample of the portion of the root or rhizome system (EL$_{sample}$);
b) exposing the sample to cell barrier disrupting conditions; and,
c) measuring the electrolyte leakage of the sample (EL$_{totai}$) after the sample has been exposed to the cell barrier disrupting conditions; and
d) calculating the comparative electrolyte leakage of the sample (CEL$_{sample}$):

$$CEL_{sample} = 100 \times \left(\frac{EL_{sample}}{EL_{total}}\right)$$

ii) applying heat treatment to the same or a different portion of the root or rhizome system of the specimen plant for a defined period of time and at a defined temperature;
iii) determining if the heat-treated portion has been killed by the heat treatment by:

a) measuring the electrolyte leakage of at least one sample of the heat-treated portion ($EL_{sample}$);
b) exposing the sample to cell barrier disrupting conditions; and
c) measuring the electrolyte leakage of the sample ($EL_{total}$) after the sample has been exposed to the cell barrier disrupting conditions; and,
d) calculating the comparative electrolyte leakage of the sample ($CEL_{sample}$):

$$CEL_{sample} = 100 \times \left( \frac{EL_{sample}}{EL_{total}} \right)$$

iv) repeating steps ii) and iii) on one or more further portions of the root or rhizome system of the specimen plant, wherein each time step ii) is repeated a different period of time and/or temperature is used, and
v) determining the optimum heat treatment by comparing the $CEL_{sample}$ values obtained for the different portions of the root or rhizome system.

Clause 51. A computer-implemented method of obtaining an assessment of the effectiveness of a lethal treatment which has been applied to a plant or a group of two or more plants of the same species or variety in accordance with a method according to any of clauses 1-34; the method comprising the steps of;

i) at a local computer, transmitting an electronic signal encoding input data representing one more individual $CEL_{sample}$ values or a mean $CEL_{sample}$ value of one or more root or rhizome samples taken from the treated plant or plants to a remote computer via a data communications network for processing; and
ii) at the same or a different local computer, receiving from the remote computer an electronic signal encoding data output representing an assessment of whether the treatment was successful in killing the plant or plants; wherein the assessment is made at the remote computer on the basis of the $CEL_{sample}$ values or mean $CEL_{sample}$ value.

Clause 52. A computer-implemented method of making an assessment of the effectiveness of a lethal treatment applied to a plant or a group of two or more plants in accordance with a method according to any of clauses 1-34; the method comprising the steps of;

i) at a computer, receiving an electronic signal encoding input data that represents one more individual $CEL_{sample}$ values or a mean $CEL_{sample}$ value of one or more root or rhizome samples obtained from the treated plant or plants; and
ii) at the computer, processing the input data to assess whether the treatment has been successful on the basis of the $CEL_{sample}$ values or mean $CEL_{sample}$ value and encoding the result of the assessment as output data.

Clause 53. The computer-implemented method of clause 52, wherein the output data is transmitted to a different computer as an electronic signal encoding the output data, or displayed as a readable representation of the output data on a display.

Clause 54. A computer-implemented method of obtaining an assessment of the effectiveness of a lethal treatment which has been applied to a plant or a group of two or more plants in accordance with a method according to any of clauses 1-34; the method comprising the steps of;

i) at a first computer, transmitting an electronic signal encoding input data representing a $CEL_{sample}$ value or mean $CEL_{sample}$ value obtained from the treated plant or plants to a remote second computer via a data communications network for processing; and
ii) at the second computer, receiving the electronic signal encoding input data and processing the input data to assess on the basis of the $CEL_{sample}$ value or mean $CEL_{sample}$ value whether the treatment has been successful and encoding the result of the assessment as output data.

Clause 55. A computer-implemented method according to clause 54, further comprising at the first computer or a third computer, receiving from the second computer the electronic signal encoding data output representing assessment of whether the treatment was successful in killing the plant or plants.
Clause 56. The method according to any of clauses 51-55, wherein the $CEL_{sample}$ value is obtained by:

a) measuring the electrolyte leakage of a root or rhizome sample ($EL_{sample}$) taken from the plant or group of plants after treatment;

b) thereafter, exposing the sample to cell barrier disrupting conditions;

c) remeasuring the electrolyte leakage of the sample ($EL_{total}$), after the sample has been exposed to the cell barrier disrupting conditions; and

d) calculating $CEL_{sample}$ as:

$$CEL_{sample} = 100 \times \left( \frac{EL_{sample}}{EL_{total}} \right).$$

Clause 57. The method according to any of clauses 51-55, wherein the mean $CEL_{sample}$ value is obtained by calculating individual $CEL_{sample}$ values for a plurality of samples of the plant or group of plants in accordance with the method of clause 56, and deriving a mean $CEL_{sample}$ value from the individual $CEL_{sample}$ values.

**Claims**

1. A method of determining the effectiveness of a lethal treatment applied to a plant or a group of two or more plants of the same plant species or variety; the method comprising assessing whether the treated plant or group of plants is dead on the basis of a comparative electrolyte leakage value ($CEL_{sample}$) of one or more root or rhizome samples taken from the treated plant or group of plants; wherein $CEL_{sample}$ is obtained by:

   a) measuring the electrolyte leakage of a root or rhizome sample ($EL_{sample}$);

   b) thereafter, exposing the sample to cell barrier disrupting conditions;

   c) measuring the electrolyte leakage of the sample ($EL_{total}$) again, after the sample has been exposed to the cell barrier disrupting conditions; and

   d) calculating $CEL_{sample}$ as:

$$CEL_{sample} = 100 \times \left( \frac{EL_{sample}}{EL_{total}} \right).$$

2. The method according to claim 1, which comprises calculating individual $CEL_{sample}$ values for a plurality of samples of the plant or group of plants; deriving a mean $CEL_{sample}$ value from the individual $CEL_{sample}$ values, and assessing whether the treated plant or group of plants is dead on the basis of the mean $CEL_{sample}$ value.

3. The method according to claim 2, wherein the plurality of samples of the plant or group of plants are obtained from roots and/or rhizomes collected from a site where the plant or group of plants are located, wherein the site is divided into multiple unit areas or unit volumes, and wherein the roots and/or rhizomes are collected from a proportion of the total unit areas or unit volumes.

4. The method according to claim 2 or claim 3, which comprises comparing the mean $CEL_{sample}$ value with a first critical value (C1) and indicating the plant or group of plants to be dead when the mean $CEL_{sample}$ is equal to or greater than C1.

5. The method according to claim 4, which comprises determining whether the mean $CEL_{sample}$ value is indicative that an Acceptable Outcome has been reached, and/or determining whether the mean $CEL_{sample}$ value is indicative that an Unacceptable Outcome has been reached, wherein an Acceptable Outcome is such that it is likely that fewer than v(a)% of roots and/or rhizomes of the plant or group of plants have a $CEL_{sample}$ value of less than C1, and wherein an Unacceptable Outcome is such that it is likely that more than v(u)% of roots and/or rhizomes of the plant or group of plants have a $CEL_{sample}$ value of less than C1, preferably wherein v(a)% is <0.01% and/or v(u)% is > 1.0%.

6. The method according to claim 4 or claim 5, wherein C1 is determined by reference to $CEL_{sample}$ values that have been obtained from a plurality of known dead reference samples taken from rhizomes or roots of the invasive plant species or variety to be treated; the critical value C1 being selected as the $CEL_{sample}$ value that identifies the dead reference samples with at least about 95% accuracy.

7. The method according to claim 4, claim 5 or claim 6, wherein C1 is a value between about 45% and about 75%; for example about 56%.

8. The method according to any preceding claim, wherein the cell barrier disrupting conditions of step b) comprise exposing the sample to at least one of sonication, ionising (e.g. gamma) ionising radiation, heat treatment, microwave radiation, radiofrequency radiation, physical attrition or a chemical agent (e.g. for altering the pH of an aqueous medium containing the sample); wherein preferably the treatment is a heat treatment; for example wherein the sample is heated to about 90 °C; optionally for at least 20 minutes.

9. The method according to any preceding claim, wherein the plant is a species selected from Japanese knotweed, Dwarf Japanese knotweed, Giant knotweed, Bohemian knotweed, Himalayan knotweed, railway yard knotweed, Rosa rugosa, rhododendron, buddleja, ferns, horsetails, brambles, nettles, bamboo and any other perennial plant.

10. A method for the treatment of a plant or group of two or more plants, the method comprising:

     i) applying a lethal treatment to the plant or group of plants; and
     ii) determining the effectiveness of the lethal treatment according to any one of claims 1 to 9.

11. The method according to claims 10, wherein the lethal treatment comprises one or more of a heat treatment, a herbicide treatment, a treatment with a chemical agent, electrical treatment, a pH treatment and/or a stem injection treatment.

12. The method of claim 10 or claim 11, wherein the effectiveness of the lethal treatment is determined by burying one or more reference portions of known viability of the same species as the plant or plants to be treated in soil with the plant or plants to be treated and assessing the status of the one or more reference portions after the treatment, and optionally during the treatment.

13. A method of validating a treatment for killing a plant having a root or rhizome system, the method comprising;

     i) determining a $CEL_{sample}$ value of at least one first sample of the root or rhizome system of a specimen of the plant by:

          a) measuring the electrolyte leakage of the first sample ($EL_{sample}$);
          b) exposing the first sample to cell barrier disrupting conditions;
          c) measuring the electrolyte leakage of the first sample ($EL_{total}$) after the sample has been exposed to the cell barrier disrupting conditions; and
          d) calculating the comparative electrolyte leakage of the first sample ($CEL_{sample}$):

$$CEL_{sample} = 100 \times \left( \frac{EL_{sample}}{EL_{total}} \right)$$

     ii) applying a treatment to the specimen;
     iii) determining the $CEL_{sample}$ of at least one second sample of the root or rhizome system of the specimen after treatment; and
     iv) comparing the $CEL_{sample}$ of the first and second samples before and after the treatment to determine the effectiveness of the treatment applied.

14. A method for optimising a heat treatment for killing a plant having a root or rhizome system, the method comprising;

     i) determining if a portion of the root or rhizome system of a specimen of the plant is viable by:

          a) measuring the electrolyte leakage of at least one sample of the portion of the root or rhizome system ($EL_{sample}$);
          b) exposing the sample to cell barrier disrupting conditions; and,
          c) measuring the electrolyte leakage of the sample ($EL_{total}$) after the sample has been exposed to the cell barrier disrupting conditions; and
          d) calculating the comparative electrolyte leakage of the sample ($CEL_{sample}$):

$$CEL_{sample} = 100 \times \left(\frac{EL_{sample}}{EL_{total}}\right)$$

ii) applying heat treatment to the same or a different portion of the root or rhizome system of the specimen plant for a defined period of time and at a defined temperature;

iii) determining if the heat-treated portion has been killed by the heat treatment by:

a) measuring the electrolyte leakage of at least one sample of the heat-treated portion ($EL_{sample}$);

b) exposing the sample to cell barrier disrupting conditions; and

c) measuring the electrolyte leakage of the sample ($EL_{total}$) after the sample has been exposed to the cell barrier disrupting conditions; and,

d) calculating the comparative electrolyte leakage of the sample ($CEL_{sample}$):

$$CEL_{sample} = 100 \times \left(\frac{EL_{sample}}{EL_{total}}\right)$$

iv) repeating steps ii) and iii) on one or more further portions of the root or rhizome system of the specimen plant, wherein each time step ii) is repeated a different period of time and/or temperature is used, and

v) determining the optimum heat treatment by comparing the $CEL_{sample}$ values obtained for the different portions of the root or rhizome system.

15. A computer-implemented method of obtaining an assessment of the effectiveness of a lethal treatment which has been applied to a plant or a group of two or more plants of the same species or variety in accordance with a method according to any of claims 1-9; the method comprising the steps of;

i) at a local computer, transmitting an electronic signal encoding input data representing one more individual $CEL_{sample}$ values or a mean $CEL_{sample}$ value of one or more root or rhizome samples taken from the treated plant or plants to a remote computer via a data communications network for processing; and

ii) at the same or a different local computer, receiving from the remote computer an electronic signal encoding data output representing an assessment of whether the treatment was successful in killing the plant or plants; wherein the assessment is made at the remote computer on the basis of the $CEL_{sample}$ values or mean $CEL_{sample}$ value.

16. A computer-implemented method of making an assessment of the effectiveness of a lethal treatment applied to a plant or a group of two or more plants in accordance with a method according to any of claims 1-9; the method comprising the steps of;

i) at a computer, receiving an electronic signal encoding input data that represents one more individual $CEL_{sample}$ values or a mean $CEL_{sample}$ value of one or more root or rhizome samples obtained from the treated plant or plants; and

ii) at the computer, processing the input data to assess whether the treatment has been successful on the basis of the $CEL_{sample}$ values or mean $CEL_{sample}$ value and encoding the result of the assessment as output data.

17. The method according of claim 15 or claim 16, wherein the $CEL_{sample}$ value is obtained by:

a) measuring the electrolyte leakage of a root or rhizome sample ($EL_{sample}$) taken from the plant or group of plants after treatment;

b) thereafter, exposing the sample to cell barrier disrupting conditions;

c) remeasuring the electrolyte leakage of the sample ($EL_{total}$), after the sample has been exposed to the cell barrier disrupting conditions; and

d) calculating $CEL_{sample}$ as:

$$CEL_{sample} = 100 \times \left(\frac{EL_{sample}}{EL_{total}}\right).$$

Fig. 1a

Fig. 1b

Fig. 1c

Fig. 2

EP 4 046 487 A1

Fig. 3

Fig. 4a

Fig. 4b

Fig. 5a

Fig. 5b

**Mean CEL$_{sample}$ after different time / temperature combinations for Site 1 lignified rhizomes.**

Fig. 6

EP 4 046 487 A1

**Mean CEL$_{sample}$ after different time / temperature combinations for Site 4 lignified rhizomes.** *Error Bars = +/- 1 SD*

Fig. 7

EP 4 046 487 A1

Fig. 8

Fig. 9b

Fig. 9a

Fig. 10

EP 4 046 487 A1

**Knotweed Viability Map**

Fig. 11

Europäisches
Patentamt

European
Patent Office

Office européen
des brevets

# EUROPEAN SEARCH REPORT

**Application Number**

EP 22 15 8322

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2006/103478 A1 (THURLOW COUNTRYSIDE MAN LTD [GB]; DOWNER HOWARD EDWARD [GB] ET AL.) 5 October 2006 (2006-10-05) * abstract; figure 1 * | 1-17 | INV. A01M21/04 |
| A,D | WO 2005/004583 A1 (BURGESS PHILIP DAVID [US]) 20 January 2005 (2005-01-20) * abstract; figure 1 * | 1-17 | |
| A | PAREPA MADALIN ET AL: "Testing for allelopathy in invasive plants: it all depends on the substrate!", BIOLOGICAL INVASIONS, SPRINGER INTERNATIONAL PUBLISHING, CHAM, vol. 18, no. 10, 16 June 2016 (2016-06-16), pages 2975-2982, XP036050642, ISSN: 1387-3547, DOI: 10.1007/S10530-016-1189-Z [retrieved on 2016-06-16] | 1-17 | |

**TECHNICAL FIELDS SEARCHED (IPC)**

A01M

The present search report has been drawn up for all claims

| Place of search | Date of completion of the search | Examiner |
|---|---|---|
| Munich | 13 July 2022 | Avramidis, Pavlos |

## ANNEX TO THE EUROPEAN SEARCH REPORT
## ON EUROPEAN PATENT APPLICATION NO.

EP 22 15 8322

This annex lists the patent family members relating to the patent documents cited in the above-mentioned European search report.
The members are as contained in the European Patent Office EDP file on
The European Patent Office is in no way liable for these particulars which are merely given for the purpose of information.

13-07-2022

| Patent document cited in search report | | Publication date | Patent family member(s) | | Publication date |
|---|---|---|---|---|---|
| WO 2006103478 | A1 | 05-10-2006 | EP | 1926381 A1 | 04-06-2008 |
| | | | GB | 2438153 A | 14-11-2007 |
| | | | WO | 2006103478 A1 | 05-10-2006 |
| WO 2005004583 | A1 | 20-01-2005 | AU | 2003297429 A1 | 28-01-2005 |
| | | | CA | 2529785 A1 | 20-01-2005 |
| | | | EP | 1643826 A1 | 12-04-2006 |
| | | | US | 2004255512 A1 | 23-12-2004 |
| | | | US | 2007033870 A1 | 15-02-2007 |
| | | | US | 2007180765 A1 | 09-08-2007 |
| | | | WO | 2005004583 A1 | 20-01-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2005004583 A1

- GB 2384158 A

**Non-patent literature cited in the description**

- **BEERLING, D. ; BAILEY, J. ; CONOLLY, A.** Fallopia Japonica (Houtt.) Ronse Decraene. *Journal of Ecology,* 1994, vol. 82 (4), 959-979
- **RPA.** *International Approaches to Japanese Knotweed in the Context of Property Sales, Final Report for Defra,* September 2020

- **MCKAY H.M.** Root electrolyte leakage and root growth potential as indicators of spruce and larch establishment. *Silva Fennica,* 1998, vol. 32 (2), 241-252